# EUROPEAN PATENT SPECIFICATION

(11) **EP 4 408 192 B1**
(45) Date of publication and mention of the grant of the patent: **18.11.2026**
(21) Application number: 22797363.3
(22) Date of filing: 30.09.2022
(51) Int. Cl.: A23L 2/52, A23L 33/10, A23L 33/12, A61K 9/00, A61K 9/107, A61K 31/201, A61K 31/706, A61P 3/04, A61P 9/10, A61P 25/08, A61P 39/00

(54) **NICOTINAMIDE RIBOSIDE TRIOLEATES CHLORIDE, COMPOSITIONS CONTAINING THIS COMPOUND, AND METHODS OF MAKING AND USING THIS COMPOUND**
NICOTINAMID-RIBOSID-TRIOLEAT-CHLORID, ZUSAMMENSETZUNGEN, DIE DIESE VERBINDUNG ENTHALTEN, UND VERFAHREN ZUR HERSTELLUNG UND VERWENDUNG DIESER VERBINDUNG
CHLORURE DE TRIOLEATE DE NICOTINAMIDE RIBOSIDE, COMPOSITIONS LE COMPRENANT ET MÉTHODES DE PRÉPARATION ET D'UTILISATION DE CE COMPOSÉ

(30) Priority: 30.09.2021 US 202163250362 P
(43) Date of publication of application: 07.08.2024
(73) Proprietor: Société des Produits Nestlé S.A., 1800 Vevey (CH); Cornell University, Ithaca, NY 14850 (US)
(72) Inventor: YAN, Bing, Bridgewater, New Jersey 08807 (US); UFHEIL, Gerhard, Chatham, New Jersey 07928 (US); WOOSTER, Timothy James, 1350 Orbe (CH); MOCO, Sofia, 1019VK AMSTERDAM (NL); ZAREI, Amin, Ithaca, New York 14850 (US); KHAZDOOZ, Leila, Ithaca, New York 14850 (US); ENAYATINOOK, Mojtaba, Troy AL, Alabama 36081 (US); MADARSHAHIAN, Sara, Ithaca, New York 14850 (US); ABBASPOURRAD, Alireza, New York, New York 14850 (US)
(74) Representative: Gordon, Kirsteen Helen
(86) International application number: PCT/EP2022/077247
(87) International publication number: WO 2023/052573

(56) References cited:
- WO-A1-2021/237193
- WO-A1-2021/244964
- WO-A2-2022/061234
- CN-A- 113 817 009
- US-A1- 2015 175 645
- US-A1- 2017 189 433
- US-A1- 2020 397 807
- CERCILLIEUX ANGELIQUE ET AL: "Balancing NAD+ deficits with nicotinamide riboside: therapeutic possibilities and limitations", CMLS CELLULAR AND MOLECULAR LIFE SCIENCES, BIRKHAUSER VERLAG, HEIDELBERG, DE, vol. 79, no. 8, 1 August 2022 (2022-08-01), XP037921877, ISSN: 1420-682X, [retrieved on 20220802], DOI: 10.1007/S00018-022-04499-5
- ZAREI AMIN ET AL: "Synthesis, Stability, and Bioavailability of Nicotinamide Riboside Trioleate Chloride", NUTRIENTS, vol. 14, no. 1, 27 December 2021 (2021-12-27), pages 113, XP055960036, DOI: 10.3390/nu14010113

## Description

### BACKGROUND

The present disclosure generally relates to a novel hydrophobic derivative of nicotinamide riboside chloride, namely, nicotinamide riboside trioleates chloride (NRTOCl). The present disclosure further relates to methods of making and using this compound and also compositions comprising this compound, for example liquid compositions such as beverages.

Nicotinamide adenine dinucleotide (NAD⁺) is a vital coenzyme in energy metabolism and mitochondrial functions by redox reactions.^{1,2} For the non-redox reactions, NAD⁺ is a crucial cofactor to regulate the activity of two essential protein families, sirtuins (SIRTs) and poly (ADP-ribose) polymerases (PARPs).³⁻⁵ The sirtuins have several key roles maintaining nuclear, mitochondrial, cytoplasmic or metabolic homeostasis. The most important roles of PARPs are repairing DNA and maintaining chromatin structure and function.³⁻⁵

During the aging process, the NAD⁺ level decreases, and this decrease causes defects in nuclear and mitochondrial functions, resulting in many age-associated pathologies.⁶⁻¹⁰ Supplementation of NAD⁺ precursors can restore NAD⁺ level and prevent many diseases of aging including neurodegenerative and cardiovascular diseases and metabolic disorders.¹¹⁻¹⁷ Recent studies show that boosting NAD⁺ can help for prevention and treatment of liver cancer treatment.¹⁸

Nicotinamide riboside (NR) is one of the most important NAD⁺ precursors that is orally available and can boost the level of NAD⁺ in mammalian cell up to two fold.^{17,19} NR is more effective than other NAD⁺ precursors, such as niacin and nicotinamide, because NR is metabolized to NAD⁺ in fewer steps (**FIG**. **1**).¹⁹

Studies have confirmed that supplementation of NR shows numerous health benefits in many animals and humans, especially middle-aged and older adults. For examples, NR supplementation can decrease DNA and mitochondria damage,²⁰ Alzheimer's disease,²¹ obesity,²²⁻²⁴ diabetes,^{24,25} muscle degeneration⁷ and aging.²⁶ Supplementation of NR not only increases lactation and nursing behaviors of new mothers but also improves the quality of milk by stimulating maternal transmission nutrients into the milk.¹³ Research also demonstrated that NR shows antiviral effect against HIV and hepatitis B.²⁶ Compositions of nicotinamide riboside analogs, including esters and carbonates, for use in elevating levels of nicotinamide adenine dinucleotide in cells and tissues of an organism and as a result preventing a disease or a condition in an organism, are disclosed in US 2017/189433 A1.

Infections caused by pathogens, specially SARS-CoV-2 and COVID-19, drastically decline the NAD⁺ levels leading to a defect in the immune response.²⁶ The use of NR helps combat COVID-19 infection by keeping the NAD⁺ levels constant leading to activate the innate immune response to fight the infection.^{26,27}

### SUMMARY

Nicotinamide riboside chloride (NRCl) is the chloride salt of NR marketed as Niagen^{™} in a capsulated form. NRCl is used as a safe nourishing supplement approved by the U.S. Food and Drug Administration (FDA) to boost the NAD⁺ level.²⁸ One of the challenges in using and storing NR is its inherent instability to hydrolysis. Structurally, NR is a quaternary ammonium salt containing a sensitive *N*-glycosidic bond that can spontaneously cleave in aqueous solution, yielding nicotinamide and D-ribose decomposition products. Consequently, ready-to-drink (RTD) beverages containing NR are difficult to develop (**FIG**. **2**).

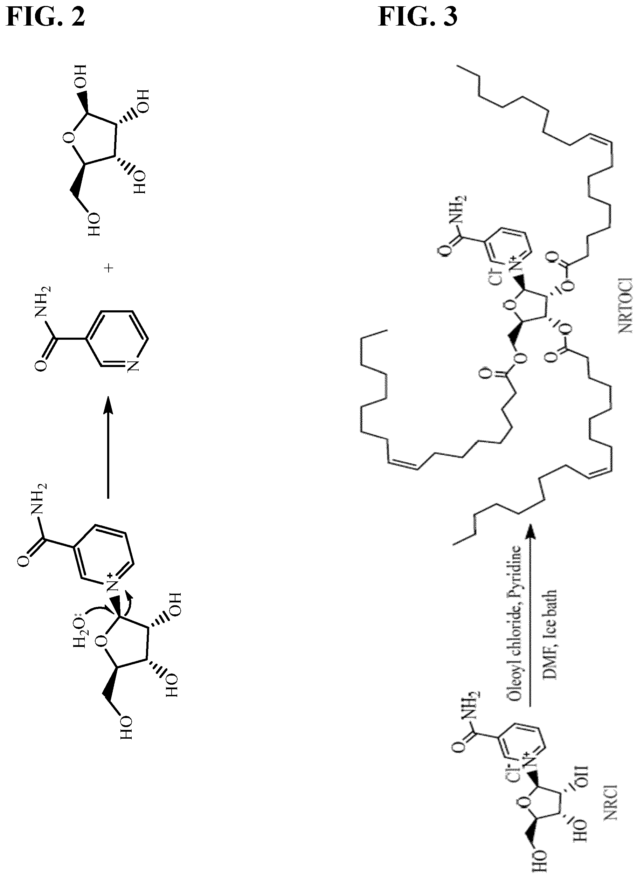

The experimental example set forth herein reports the synthesis of nicotinamide riboside trioleates chloride (NRTOCl) as a novel hydrophobic NRCl derivative by the reaction of NRCl and oleoyl chloride (**FIG. 3**). Contrary to NRCl, this new compound is not soluble in water but is easily dissolved in canola, corn, and medium chain triglycerides (MCT) oil at room temperature. The stability of NRCl and NRTOCl in water at 35 °C was studied, and the results confirmed eighty-eight (88) times more stability of NRTOCl than that of NRCl.

NRTOCl was easily dissolved in canola oil, so an oil-in-water emulsion was made by dissolving NRTOCl in canola oil in the presence of sodium caseinate as a food grade emulsifier. In this emulsion, the stability of NRTOCl enormously increased, so that at a temperature of 35 °C, the NRTOCl was 213 times more stable in emulsion than NRCl under the same conditions. Finally, the bioavailability of NRTOCl was investigated by studying its digestibility in a simulated intestinal phase. The results demonstrate that NRTOCl is digestible (e.g., 1-10% or even 1-20%) to release NR in the presence of porcine pancreatin in the simulated intestinal phase. These obtained results show that NRTOCl can be potentially be used as an NR booster in beverages such as ready-to-drink (RTD) beverages.

Furthermore, the specific use of the long chain fatty acid in NRTOCl is advantageous over the short chain fatty acid in nicotinamide riboside tributyrates chloride (NRTBCl) which did not demonstrate good solubility as shown in an additional experimental example.

Additional features and advantages are described herein and will be apparent from the following Figures and Detailed Description.

### BRIEF DESCRIPTION OF DRAWINGS

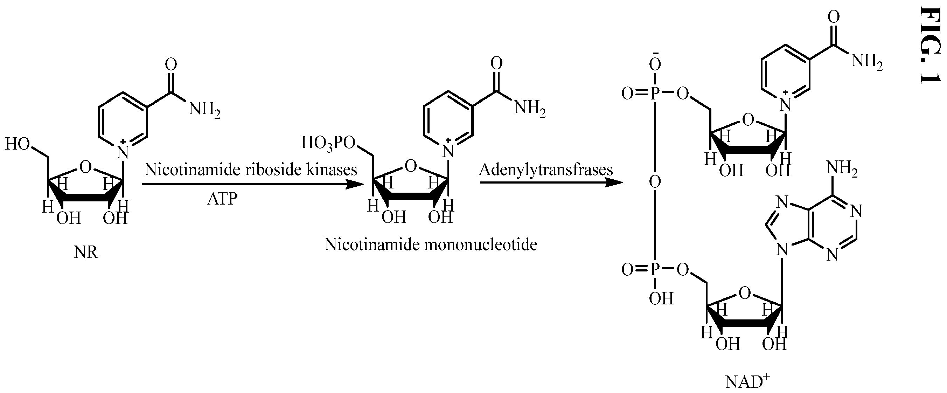

**FIG. 1** is a schematic diagram generally illustrating conversion of NR into NAD⁺ in mammalian cell.
**FIG. 2** is a schematic diagram generally illustrating hydrolysis of NR.
**FIG. 3** is a schematic diagram generally illustrating synthesis of NRTOCl using oleoyl chloride, as disclosed herein.
**FIG. 4** is a graph showing results from the experimental example disclosed herein, specifically, FT-IR of NRTOCl .
**FIG. 5** is a graph showing results from the experimental example disclosed herein, specifically, ¹H NMR of NRTOCl in CDCl₃.
**FIG. 6** is graphs showing results from the experimental example disclosed herein, specifically, Expanded ¹H NMR of NRTOCl .

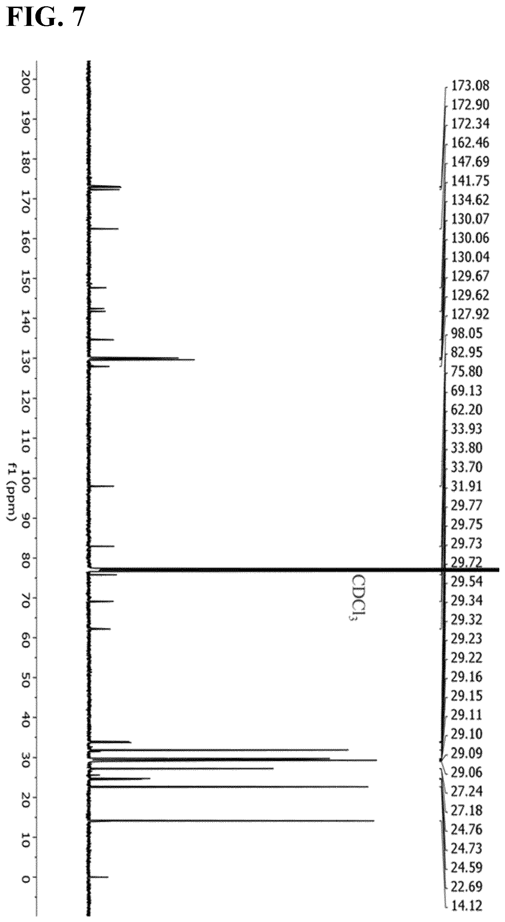

**FIG. 7** is a graph showing results from the experimental example disclosed herein, specifically, ¹³C NMR of NRTOCl in CDCl₃.
**FIG. 8** is graphs showing results from the experimental example disclosed herein, specifically, Expanded ¹³C NMR of NRTOCl.
**FIGS. 9A** and **9B** are graphs showing results from the experimental example disclosed herein, specifically, SRM LC-MS of NRTOCl. **FIG. 9A** is SRM LC of NRTOCl, and **FIG. 9B** is mass spectrum of NRTOCl.
**FIGS. 10A, 10B** and **10C** are photographs showing results from the experimental example disclosed herein. **FIG. 10A** is NRTOCl dispersed in water, and **FIGS. 10B** and **10C** are transmission electron microscopy (TEM) images of NRTOCl dispersed in water.
**FIG. 11** is a graph showing results from the experimental example disclosed herein, specifically, stability of NRTOCl and NRCl in DI water at 35 °C.
**FIG. 12** is a schematic diagram generally illustrating hydrolysis of NRTO⁺ and NR⁺ from *N*-glycoside bond.
**FIG. 13** is a schematic diagram generally illustrating hydrolysis of NRTOCl nanoparticles in the outer layer in contact with water when NRTOCl is dispersed in water.

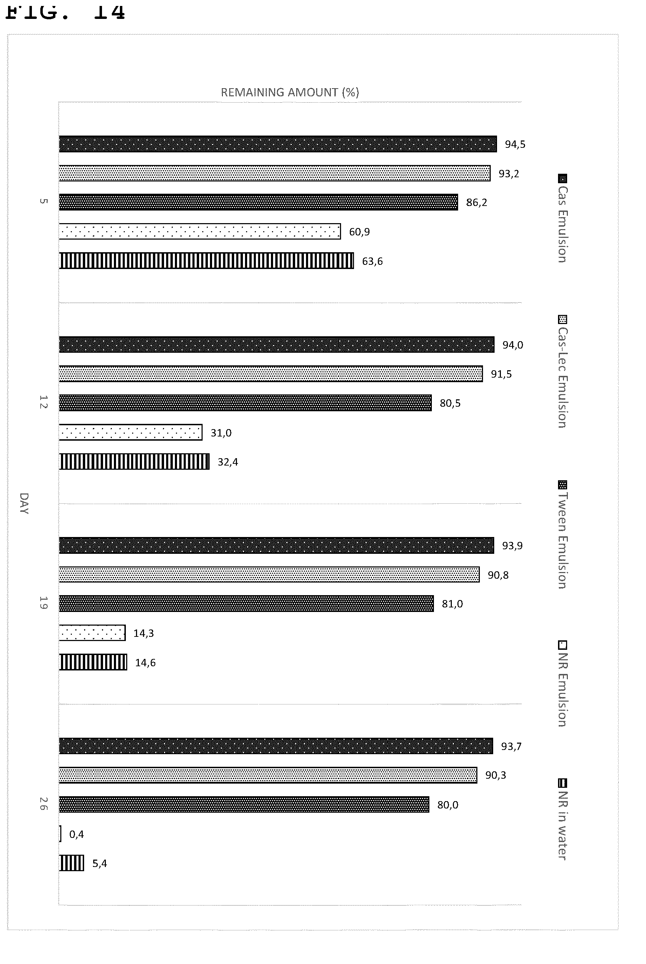

**FIG. 14** is a graph showing results from the first experimental example disclosed herein, specifically, comparison of hydrolysis stability of NRTOCl emulsions with NRCl emulsion or NRCl in water at 35 °C for 26 days.
**FIG. 15** is a graph showing results from the first experimental example disclosed herein, specifically, comparison of hydrolysis stability of NRTOCl emulsions with NRCl in water at 25 °C for 42 days.
**FIG. 16** is a schematic diagram generally illustrating the digestion of NRTO⁺ in the simulated intestinal phase.
**FIGS. 17A** and **17B** are graphs showing results from the first experimental example disclosed herein, specifically, SRM LC-MS of released NRCl. **FIG. 17A** is SRM LC of released NRCl, and **FIG. 17B** is mass spectrum of released NRCl.
**FIG. 18** shows the FT-IR of nicotinamide riboside tributyrates chloride (NRTBCl) from the second experimental example disclosed herein.
**FIG. 19** shows the ¹H NMR of NRTBCl in CDCl₃ from the second experimental example disclosed herein.
**FIG. 20** shows the ¹³C NMR of NRTBCl in CDCl₃ from the second experimental example disclosed herein.
**FIGS. 21A** and **21B** show the SRM LC-MS of NRTBCl from the second experimental example disclosed herein. **FIG. 21A** shows SRM LC of NRTBCl, and **FIG. 21B** shows mass spectrum of NRTBCl.
**FIGS. 22A, 22B and 22C** show size measurement of NRTBCl in water from the second experimental example disclosed herein.
**FIG. 23** shows the stability of NRTBCl in MilliQ (MQ) water at 35 °C for 6 days from the second experimental example disclosed herein.

### DETAILED DESCRIPTION

### Definitions

Some definitions are provided hereafter. Nevertheless, definitions may be located in the "Embodiments" section below, and the above header "Definitions" does not mean that such disclosures in the "Embodiments" section are not definitions.

All percentages expressed herein are by weight of the total weight of the composition unless expressed otherwise. As used herein, "about," "approximately" and "substantially" are understood to refer to numbers in a range of numerals, for example the range of -10% to +10% of the referenced number, preferably -5% to +5% of the referenced number, more preferably -1% to +1% of the referenced number, most preferably -0.1% to +0.1% of the referenced number. All numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used in this disclosure and the appended claims, the singular forms "a," "an" and "the" include plural referents unless the context clearly dictates otherwise. Thus, for example, reference to "a vitamin" or "the vitamin" encompass both an embodiment having a single vitamin and an embodiment having two or more vitamins.

The words "comprise," "comprises" and "comprising" are to be interpreted inclusively rather than exclusively. Likewise, the terms "include," "including" and "or" should all be construed to be inclusive, unless such a construction is clearly prohibited from the context. Nevertheless, the compositions disclosed herein may lack any element that is not specifically disclosed herein. Thus, a disclosure of an embodiment using the term "comprising" includes a disclosure of embodiments "consisting essentially of" and "consisting of" the components identified.

The terms "at least one of" and "and/or" used in the respective context of "at least one of X or Y" and "X and/or Y" should be interpreted as "X," or "Y," or "X and Y." For example, "at least one of sodium caseinate or lecithin" and "sodium caseinate and/or lecithin" should be interpreted as "sodium caseinate without lecithin," or "lecithin without sodium caseinate," or "both sodium caseinate and lecithin."

Where used herein, the terms "example" and "such as," particularly when followed by a listing of terms, are merely exemplary and illustrative and should not be deemed to be exclusive or comprehensive. As used herein, a condition "associated with" or "linked with" another condition means the conditions occur concurrently.

"Prevention" includes reduction of risk, incidence and/or severity of a condition or disorder. The terms "treatment" and "treat" include treatments that slow the development of a targeted pathologic condition or disorder and also curative, therapeutic or disease-modifying treatment, including therapeutic measures that cure, slow down, lessen symptoms of, and/or halt progression of a diagnosed pathologic condition or disorder; and treatment of patients at risk of contracting a disease or suspected to have contracted a disease, as well as patients who are ill or have been diagnosed as suffering from a disease or medical condition. The terms "treatment" and "treat" do not necessarily imply that a subject is treated until total recovery. The terms "treatment" and "treat" are also intended to include the potentiation or otherwise enhancement of one or more primary prophylactic or therapeutic measures. As non-limiting examples, a treatment can be performed by a patient, a caregiver, a doctor, a nurse, or another healthcare professional.

As used herein, a prophylactically or therapeutically "effective amount" is an amount that prevents a deficiency, treats a disease or medical condition in an individual, or, more generally, reduces symptoms, manages progression of the disease, or provides a nutritional, physiological, or medical benefit to the individual. The relative terms "promote," "improve," "increase," "enhance" and like terms refer to superiority of the composition disclosed herein (which comprises NRTOCl) and its properties and effects, relative to the properties and effects of a composition using nicotinamide riboside chloride instead of NRTOCl but otherwise identically formulated.

As used herein, the terms "food," "food product" and "food composition" mean a product or composition that is intended for oral ingestion by a human or other mammal and comprises at least one nutrient for the human or other mammal.

"Nutritional compositions" and "nutritional products," as used herein, include any number of food ingredients and possibly optional additional ingredients based on a functional need in the product and in full compliance with all applicable regulations. The optional ingredients may include, but are not limited to, conventional food additives, for example one or more, acidulants, additional thickeners, buffers or agents for pH adjustment, chelating agents, colorants, emulsifiers, excipients, flavor agents, mineral, osmotic agents, a pharmaceutically acceptable carrier, preservatives, stabilizers, sugar, sweeteners, texturizers, and/or vitamins. The optional ingredients can be added in any suitable amount.

The term "unit dosage form," as used herein, refers to physically discrete units suitable as unitary dosages for human and animal subjects, each unit containing a predetermined quantity of the composition disclosed herein in an amount sufficient to produce the desired effect, optionally in association with a pharmaceutically acceptable diluent, carrier or vehicle. The specifications for the unit dosage form depend on the particular compounds employed, the effect to be achieved, and the pharmacodynamics associated with each compound in the host.

A "subject" or "individual" is a mammal, preferably a human. The term "elderly" in the context of a human means an age from birth of at least 60 years, preferably above 63 years, more preferably above 65 years, and most preferably above 70 years. The term "older adult" in the context of a human means an age from birth of at least 45 years, preferably above 50 years, more preferably above 55 years, and includes elderly individuals.

### Embodiments

Aspects of the present disclosure include nicotinamide riboside trioleates chloride (NRTOCl) and compositions comprising NRTOCl, for example liquid compositions such as beverages. The composition can be a food product or other nutritional composition formulated for oral administration. The composition can comprise an emulsion in which the NRTOCl is dispersed; for example, the emulsion can comprise an oil phase in which at least a portion of the NRTOCl is dispersed. In some embodiments, the oil comprises at least one of canola oil, corn oil or MCT oil. In some embodiments, the oil phase further comprises an emulsifier, for example at least one of sodium caseinate or lecithin (preferably both in a particularly preferred embodiment).

Another aspect is a composition for use in a therapeutic method of promoting an increase of intracellular levels of nicotinamide adenine dinucleotide (NAD⁺) in cells and tissues, e.g., for improving cell and tissue survival, by administering NRTOCl (e.g., an effective amount thereof) or a composition comprising NRTOCl (e.g., an effective amount thereof) to an individual. The individual can be an older adult or elderly

Yet another aspect is a composition for use in a method of decreasing at least one of DNA damage or mitochondria damage and/or treating or preventing at least one condition selected from the group consisting of (a) a neurodegenerative condition; (b) overweight or obesity; (c) a cardiovascular disease such as heart disease; (d) one or more of diabetes, hyperinsulinemia, an insulin resistance disorder, or insulin insensitivity; (d) muscle degeneration; (e) a disease or disorder associated with aging; (f) a viral infection such as HIV, hepatitis B, SARS-CoV-2 or COVID-19; (g) stress; (h) a blood clotting disorder; (i) inflammation; (j) cancer; (k) an eye disorder; and (l) flushing. The method comprises administering NRTOCl (e.g., an effective amount thereof) or a composition comprising NRTOCl (e.g., an effective amount thereof) to a subject in need thereof or at risk thereof.

The term "neurological condition" refers to a disorder of the nervous system. Neurological conditions may result from damage to the brain, spinal column or nerves, caused by illness or injury. Non-limiting examples of the symptoms of a neurological condition include paralysis, muscle weakness, poor coordination, loss of sensation, seizures, confusion, pain and altered levels of consciousness. An assessment of the response to touch, pressure, vibration, limb position, heat, cold, and pain as well as reflexes can be performed to determine whether the nervous system is impaired in a subject.

Some neurological conditions are life-long, and the onset can be experienced at any time. Other neurological conditions, such as cerebral palsy, are present from birth. Some neurological conditions, such as Duchenne muscular dystrophy, commonly appear in early childhood, while other neurological conditions, such as Alzheimer's disease and Parkinson's disease, affect mainly older people. Some neurological conditions have a sudden onset due to injury or illness, such as a head injury or stroke, or cancers of the brain and spine.

In an embodiment, the neurological condition is the result of traumatic damage to the brain. Additionally, or alternatively, the neurological condition is the result of an energy deficiency in the brain or in the muscles.

Examples of neurological conditions include migraine, memory disorder, age-related memory disorder, brain injury, neurorehabilitation, stroke and post-stroke, amyloid lateral sclerosis, multiple sclerosis, cognitive impairment, mild cognitive impairment (MCI), cognitive impairment post-intensive care, age-induced cognition impairment, Alzheimer's disease, Parkinson's disease, Huntingdon's disease, inherited metabolic disorders (such as glucose transporter type 1 deficiency syndrome and pyruvate dehydrogenase complex deficiency), bipolar disorder, schizophrenia, and/or epilepsy.

It may be appreciated that the compounds, compositions and methods of the present invention may be beneficial to prevent and/or treat neurological conditions listed above, in particular, to maintain or improve brain or nervous system function.

"Diabetes" refers to high blood sugar or ketoacidosis, as well as chronic, general metabolic abnormalities arising from a prolonged high blood sugar status or a decrease in glucose tolerance. "Diabetes" encompasses both the type I and type II (Non Insulin Dependent Diabetes Mellitus or NIDDM) forms of the disease. The risk factors for diabetes may include but are not limited to the following factors: waistline of more than 40 inches for men or 35 inches for women, blood pressure of 130/85 mmHg or higher, triglycerides above 150 mg/dl, fasting blood glucose greater than 100 mg/dl or high-density lipoprotein of less than 40 mg/dl in men or 50 mg/dl in women.

The term "hyperinsulinemia" refers to a state in an individual in which the level of insulin in the blood is higher than normal.

The term "insulin resistance" refers to a state in which a normal amount of insulin produces a subnormal biologic response relative to the biological response in a subject that does not have insulin resistance.

An "insulin resistance disorder," as discussed herein, refers to any disease or condition that is caused by or contributed to by insulin resistance. Examples include: diabetes, obesity, metabolic syndrome, insulin-resistance syndromes, syndrome X, insulin resistance, high blood pressure, hypertension, high blood cholesterol, dyslipidemia, hyperlipidemia, dyslipidemia, atherosclerotic disease including stroke, coronary artery disease or myocardial infarction, hyperglycemia, hyperinsulinemia and/or hyperproinsulinemia, impaired glucose tolerance, delayed insulin release, diabetic complications, including coronary heart disease, angina pectoris, congestive heart failure, stroke, cognitive functions in dementia, retinopathy, peripheral neuropathy, nephropathy, glomerulonephritis, glomerulosclerosis, nephrotic syndrome, hypertensive nephrosclerosis some types of cancer (such as endometrial, breast, prostate, and colon), complications of pregnancy, poor female reproductive health (such as menstrual irregularities, infertility, irregular ovulation, polycystic ovarian syndrome (PCOS)), lipodystrophy, cholesterol related disorders, such as gallstones, cholescystitis and cholelithiasis, gout, obstructive sleep apnea and respiratory problems, osteoarthritis, and prevention and treatment of bone loss, e.g. osteoporosis.

"Overweight" individuals have a body mass index (BMI) of at least 25 or greater, and "obese" individuals have a body mass index (BMI) of at least 30 or greater. Overweight and obesity may or may not be associated with insulin resistance.

In some embodiments, a method of treating or preventing cancer comprises administering NRTOCl (e.g., an effective amount thereof) or a composition comprising NRTOCl (e.g., an effective amount thereof) to a subject in need thereof or at risk thereof, for example by inhibiting inosine 5'-monophosphate dehydrogenase and/or reducing the amount of NAD+ in the cells comprising the cancer.

"Cancer" means any of various cellular diseases with malignant neoplasms characterized by the proliferation of anaplastic cells. It is not intended that the diseased cells must actually invade surrounding tissue and metastasize to new body sites. Cancer can involve any tissue of the body and have many different forms in each body area. Most cancers are named for the type of cell or organ in which they start.

The cancer can be selected from the group consisting of pancreatic cancer; endometrial cancer; small cell and non-small cell cancer of the lung (including squamous, adneocarcinoma and large cell types); squamous cell cancer of the head and neck; bladder, ovarian, cervical, breast, renal, CNS, and colon cancers; myeloid and lymphocyltic leukemia; lymphoma; heptic tumors; medullary thyroid carcinoma; multiple myeloma; melanoma; retinoblastoma; and sarcomas of the soft tissue and bone. Optionally the NRTOCl is administered in combination with another chemotherapeutic agent, for example in the same composition.

Another aspect is a non-therapeutic method, or a composition for use in a therapeutic method, which is a method of reducing the weight of a subject, or preventing weight gain in a subject. The method comprises administering NRTOCl (e.g., an effective amount thereof) or a composition comprising NRTOCl (e.g., an effective amount thereof) to a subject in need thereof or at risk thereof.

Yet another aspect is a composition for use in a method of treating or preventing drug toxicity and/or an adverse drug reaction. The method comprises administering NRTOCl (e.g., an effective amount thereof) or a composition comprising NRTOCl (e.g., an effective amount thereof) to a subject in need thereof or at risk thereof, for example a subject concurrently administered a drug such as a statin.

"Adverse drug reaction" means any response to a drug that is noxious and unintended and occurs in doses for prophylaxis, diagnosis, or therapy including side effects, toxicity, hypersensitivity, drug interactions, complications, or other idiosyncrasy. Side effects are often adverse symptom produced by a therapeutic serum level of drug produced by its pharmacological effect on unintended organ systems (e.g., blurred vision from anticholinergic antihistamine). A toxic side effect is an adverse symptom or other effect produced by an excessive or prolonged chemical exposure to a drug (e.g., digitalis toxicity, liver toxicity). Hypersensitivities are immune-mediated adverse reactions (e.g., anaphylaxis, allergy). Drug interactions are adverse effects arising from interactions with other drugs, foods or disease states (e.g., warfarin and erythromycin, cisapride and grapefruit, loperamide and *Clostridium difficile* colitis). Complications are diseases caused by a drug (e.g., NSAID-induced gastric ulcer, estrogen-induced thrombosis). The adverse drug reaction may be mediated by known or unknown mechanisms (e.g., Agranulocytosis associated with chloramphenicol or clozapine). Such adverse drug reaction can be determined by subject observation, assay or animal model well-known in the art.

In an embodiment, NRTOCl is used to decrease a level and/or an activity of a sirtuin protein and may be administered with one or more of the following compounds: nicotinamide (NAM), suranim; NF023 (a G-protein antagonist); NF279 (a purinergic receptor antagonist); Trolox (6-hydroxy-2,5,7,8, tetramethylchroman-2-carboxylic acid); (-)-epigallocatechin (hydroxy on sites 3,5,7,3',4',5'); (-)-epigallocatechin gallate (Hydroxy sites 5,7,3',4',5' and gallate ester on 3); cyanidin choloride (3,5,7,3',4'-pentahydroxyflavylium chloride); delphinidin chloride (3,5,7,3',4',5'-hexahydroxyflavylium chloride); myricetin (cannabiscetin; 3,5,7,3',4',5'-hexahydroxyflavone); 3,7,3',4',5'-pentahydroxyflavone; gossypetin (3,5,7,8,3',4'-hexahydroxyflavone), sirtinol; and splitomicin.

The NRTOCl may be administered to humans or animals, in particular companion animals, pets or livestock. It has beneficial effects for any age group. Preferably, the composition is formulated for administration to infants, juveniles, adults or elderly.

Preferably the NRTOCl is orally administered to the individual in a beverage, and the unit dosage form is a predetermined amount of the beverage (e.g., a predetermined amount of the beverage that comprises an effective amount of NRTOCl).

In some embodiments, the supplement can be a ready to drink (RTD) beverage in a container, and the unit dosage form is a predetermined amount of the RTD beverage sealed in the container, which is opened for the oral administration. For example, the predetermined amount of the RTD beverage can comprise an effective amount of NRTOCl. An RTD beverage is a liquid that can be orally consumed without addition of any further ingredients.

In other embodiments, the method comprises forming the beverage by reconstituting a unit dosage form of a powder, which comprises the NRTOCl, in a diluent such as water or milk to thereby form the beverage subsequently orally administered to the individual (e.g., within about ten minutes after reconstitution, within about five minutes after reconstitution, or within about one minute after reconstitution). The unit dosage form of the powder can be sealed in a sachet or other package, which can be opened for the reconstitution and subsequent oral administration.

The unit dosage form of the supplement can contain excipients, emulsifiers, stabilizers and mixtures thereof.

The NRTOCl can be administered at least one day per week, preferably at least two days per week, more preferably at least three or four days per week (e.g., every other day), most preferably at least five days per week, six days per week, or seven days per week. The time period of administration can be at least one week, preferably at least one month, more preferably at least two months, most preferably at least three months, for example at least four months.

In an embodiment, dosing is at least daily; for example, a subject may receive one or more doses daily. In some embodiments, the administration continues for the remaining life of the individual. In other embodiments, the administration occurs until no detectable symptoms of the medical condition remain. In specific embodiments, the administration occurs until a detectable improvement of at least one symptom occurs and, in further cases, continues to remain ameliorated.

In view of the preceding disclosures, an embodiment provided herein is NRTOCl. Another embodiment is a composition comprising NRTOCl and optionally at least one of protein, lipid, carbohydrate, vitamin or mineral. In some embodiments, the composition is formulated for oral administration, and preferably is a beverage, such as a Ready-to-Drink (RTD) beverage sealed in a container or a powder formulated for reconstitution in a diluent to form a reconstituted beverage.

Another embodiment is a unit dosage form of a composition comprising NRTOCl, the unit dosage form comprising an amount of the NRTOCl that is therapeutically or prophylactically effective for an individual to whom the unit dosage form is administered.

Yet another embodiment is a method of making a composition, the method comprising adding NRTOCl to at least one other component. In some embodiments, the composition is formulated for oral administration, and the at least one other component is edible.

In some embodiments of these methods, the composition is an emulsion, preferably an oil-in-water emulsion comprising an oil phase in which at least a portion of the NRTOCl is dispersed and optionally in which an emulsifier such as at least one of sodium caseinate or lecithin is dispersed. The oil phase can comprise at least one of canola oil, corn oil, or medium chain triglyceride (MCT) oil, in which at least a portion of the NRTOCl is dispersed.

In some embodiments of these methods, the composition is administered daily to the individual for at least one week. In some embodiments of these methods, the individual is selected from the group consisting of a human infant, a human child, a human adolescent, a human adult, an elderly human, and an animal such as a companion animal.

In some embodiments of these methods, the composition is orally administered, preferably as a beverage, more preferably a Ready-to-Drink (RTD) beverage sealed in a container which is opened before administration or a powder formulated for reconstitution in a diluent to form a reconstituted beverage before administration.

### EXAMPLE

The following non-limiting examples generally illustrate the concepts underlying the embodiments disclosed herein.

### Example 1

### 1. Experimental section

### 1.1. General

Materials: Nicotinamide riboside chloride (beta form) was a gift from ChromaDex Company. Oleoyl chloride was purchased from Aldrich with 89% purity, silica gel (P60, 40-63 µm, 60 Å) was purchased from SiliCycle and Silica Gel 60 F254 Coated Aluminum-Backed TLC Sheets were purchased from EMD Millipore (Billerica, MA, USA). Bovine bile (B3883) and pancreatin from porcine pancreas (P7545, 8 × USP) were purchased from Aldrich.

Characterization: A 500 NMR (Bruker INOVA) spectrometer was used to prepare the ¹H and ¹³C-NMR spectra in CDCl₃. Fourier transform infrared spectra (FTIR) were recorded on a Shimadzu IRAffinity-1S spectrophotometer by collecting 128 scans with a resolution of 8 cm⁻¹. Ultraviolet-visible (UV-vis) spectroscopy was recorded on a Shimadzu UV-2600 spectrophotometer. An Agilent 1200 LC System equipped with Binary SL Pump & Diode Array Detector and a Shodex RI-501 Refractive Index Detector (single channel) was used to perform the high-performance liquid chromatography (HPLC) measurements.

The HPLC was equipped with an ultraviolet detector (HPLCUV). Reversed-phase HPLC was performed on a Luna 100 Å (150 mm × 4.6 mm), and the column temperature was set at 25 °C. The injection volume was 10.0 µL and ammonium acetate (20 mM) was used as the mobile phase with a flow rate of 0.7 mL min⁻¹ over 45 or 60 min. All samples were filtrated using a 13 mm Nylon syringe filter with a 0.22 µm pore size before measurement.

LC-MS analysis used LC (Agilent 1100 series) coupled with a mass spectrometer. Reverse-phase chromatography was used with a Phenomenex Luna Omega (Phenomenex) LC column with the following specifications: 100 × 4.6 mm, 3 µm, polar C18, 100 Å pore size with a flow rate of 0.3 mL min⁻¹. LC eluents include MilliQ-water (solvent A) and acetonitrile (solvent B) using gradient elution (solution A:B composition change with time: 0 min: 95:5, 3 min: 95:5, 15 min: 85:15, 17 min: 90:10, and 20 min 95:5).

The mass spectrometer (Finnigan LTQ mass spectrometer) was equipped with an electrospray interface (ESI) set in positive electrospray ionization mode for analyzing the NRTOCl, NRCl and nicotinamide. The optimized parameters were a sheath gas flow rate at 20 arbitrary unit, spray voltage set at 4.00 kV, capillary temperature at 350 °C, capillary voltage at 41.0 V, and tube lens set at 125.0 V.

The particle size distribution, mean particle diameter (Zeta average size) and zeta-potential of NRTOCl in DI water were measured by using a commercial dynamic light-scattering device (Nano-ZS, Malvern Instruments, Worcestershire, UK). Tecnai F20 TEM/STEM transmission electron microscope (200 kV) was used for characterizing the structure and morphology of prepared NRTOCl nanoparticles.

### 1.2. General procedure for the synthesis of NRTOCl

To a round bottom flask in ice bath, 200 mg of NRCl, 0.55 mL of pyridine and 4.75 mL of DMF were added. Then, 2.0 mL of oleoyl chloride was dropwise added, and the reaction mixture was stirred for 3 hours under nitrogen blanket. The progress of the reaction was followed by TLC. After 3 hours, 5 mL of methanol was added to the reaction mixture to neutralize the extra amount of oleoyl chloride, and after that, the solvent was evaporated using a rotary evaporator under reduced pressure. The crude product was extracted in hexane and finally purified using column chromatography on SiO₂. Eluent was the mixture of CH₃OH (12%) and EtOAc (88%). The purified NRTOCl was obtained in 64.3% (479.2 mg) as a pale-cream-colored greasy product (λₘₐₓ in methanol was 267 nm).

### 1.3. Preparation of 15 wt.% NRTOCl in canola oil as a stock for making oil-in-water emulsions

2154 mg of canola oil was added to a 15 mL falcon tube containing 380 mg of NRTOCl. Then, the tube was placed in a water bath and was shaken at around 35 °C until NRTOCl was completely dissolved in canola oil. After dissolving NRTOCl in canola oil, the sample was kept at room temperature for the next studies. During the storage of NRTOCl in canola oil at room temperature, the solution was stable and clear without any sediment.

### 1.4. Preparation of aqueous phase for making NRTOCl in oil-in-water emulsion using Na-caseinate (2 wt.%), KCl (0.3 wt.%), NaCl (0.1 wt.%), CaCl₂ (0.2 wt.%) and NaN₃ (0.01 wt.%)

2 g of sodium caseinate was gradually added to 100 mL of DI water at 70 °C, and the mixture was stirred for 5 minutes. Then, the temperature was increased to 75 °C for 10 minutes. After cooling the solution to 50 °C, 0.3 g of KCl, 0.1 g of NaCl and 0.01 g of NaN₃ were added to the mixture and stirred for 2 minutes. Subsequently, 0.2 g of CaCl₂ was gradually added to this solution and stirred for five (5) extra minutes. After decreasing the temperature to 25 °C, the mixture was diluted to 100 mL by adding DI water and homogenized at 10,000 rpm for 2 minutes.

### 1.5 Preparation of NRTOCl and NRCl oil-in-water emulsions for stability study

Three different types of NRTOCl oil-in-water emulsions were made according to the following processes:

### 1.5.1. Cas emulsion:

The emulsion was prepared using 480 mg of oil stock containing 15 wt.% NRTOCl in canola oil and an aqueous phase (14.52 g) of Na-caseinate (2 wt.%), KCl (0.3 wt.%), NaCl (0.1 wt.%), CaCl₂ (0.2 wt.%) and NaN₃ (0.01 wt.%). The oil phase was added to the aqueous phase and homogenized at room temperature at 16,800 rpm for 150 seconds.

### 1.5.2. Cas-Lec emulsion:

The emulsion was -prepared using 480 mg of oil stock containing 15 wt.% NRTOCl in canola oil and 0.025 g of lecithin. The aqueous phase (14.5 g) contained Na-caseinate (2 wt.%), KCl (0.3 wt.%), NaCl (0.1 wt.%), CaCl₂ (0.2 wt.%) and NaN₃ (0.01 wt.%). The oil phase was added to the aqueous phase and homogenized at room temperature at 16,800 rpm for 150 seconds.

### 1.5.3. Tween emulsion:

The emulsion was -prepared using 480 mg of oil stock containing 15 wt.% NRTOCl in canola oil and an aqueous phase (14.52 g) of polysorbate 80 (Tween 80) (2 wt.%). The oil phase was added to the aqueous phase and homogenized at room temperature at 16,800 rpm for 150 seconds.

### 1.5.4. NR emulsion:

The emulsion was prepared using around 480 mg of canola oil in an aqueous phase (14.52 g) of NRCl (19 mg), Na-caseinate (2 wt.%), KCl (0.3 wt.%), NaCl (0.1 wt.%), CaCl₂ (0.2 wt.%) and NaN₃ (0.01 wt.%). The oil phase was added to the aqueous phase and homogenized at room temperature at 16,800 rpm for 150 seconds.

### 1.6. Sample preparation of NRTOCl and NRCl emulsions for determination of released nicotinamide during the stability study

After each specific time of stability for NRTOCl and NRCl emulsions, 1.5 mL of each emulsion was centrifuged (14,000 rpm) for 20 minutes at room temperature. Then, the aqueous phase was separated and filtered by a 0.22 µm filter for determination of released nicotinamide by HPLC analysis.

### 1.7. In vitro digestion study

*In vitro* digestion of NRTOCl in pure form and dissolved in MCT oil was investigated in the following procedures.

### 1.7.1. In vitro digestion study of pure NRTOCl dispersed in simulated intestinal phase

The buffer for simulated intestinal phase was prepared according to the following protocol.²⁹ Specifically, 60 mg of NRTOCl was dissolved in 0.3 mL ethanol and added to 10 g of a buffer solution containing 400 mg bile bovine. 0.75 mL of a CaCl₂ solution (0.3 M) was added to this mixture, and the pH was adjusted around 7 by using HCl (1 M). Then, 400 mg of fresh porcine pancreatin was dispersed in 4 mL of buffer solution and added to the mixture. The sample was placed in an incubator (250 rpm) at 37 °C for 30 minutes. Subsequently, the sample was taken out of the incubator to adjust the pH to around 7 and returned to the incubator for another 30 minutes. The incubation steps and adjusting pH were repeated every 30 minutes until 2 hours were over. For quenching the enzyme, the sample was placed in an ice bath, and then 1.5 mL of the sample was centrifuged at 14,000 rpm for 10 minutes. Finally, the aqueous phase was separated and filtered by a 0.22 µm filter for determination of released NR and nicotinamide.

### 1.7.2. In vitro digestion study of NRTOCl in MCT oil in simulated intestinal phase

60 mg of NRTOCl was dissolved in 150 mg of MCT oil to prepare 29% w/w NRTO in MCT oil. Aqueous phase was 10 g of the buffer solution containing 400 mg bile bovine. The oil phase was added to the aqueous phase and homogenized at 150,00 rpm for 150 seconds at room temperature. 0.75 mL of a CaCl₂ solution (0.3 M) was added to this mixture, and the pH was adjusted around 7 by using HCl (1 M). Then, 400 mg of fresh porcine pancreatin was dispersed in 5 mL of buffer solution and added to the mixture. The sample was placed in an incubator (250 rpm) at 37 °C for 30 minutes. Then, the sample was taken out of the incubator to adjust the pH to around 7 (by NaOH 1 M) and returned to the incubator for another 30 minutes. The incubating and adjusting pH steps were repeated every 30 minutes until 2 hours were over. For quenching the enzyme, the sample was placed in an ice bath and then 1.5 mL of the sample was centrifuged at 14000 rpm for 10 min. Finally, the aqueous phase was separated and filtered by a 0.22 µm filter for determination of released NR and nicotinamide.

### 2. Results and discussion

The present work increased the hydrolysis stability of NR by increasing its hydrophobicity with chemical modification as a fatty ester derivative. For this purpose, NRTOCl was synthesized as a new compound by the reaction between NRCl and oleoyl chloride. The best result was obtained when the reaction was carried out in DMF as a solvent and with the use of pyridine as a base. After purification of NRTOCl by column chromatography on SiO₂, the pure product was characterized by FTIR, ¹H NMR, ¹³C NMR and LC-MS.

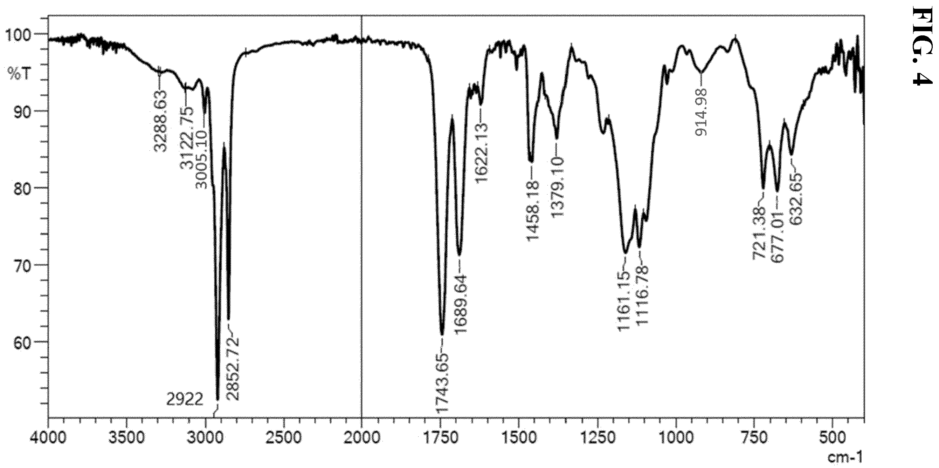

First, to determine the functional groups in structure of NRTOCl, FTIR spectrum of this compound was studied (**FIG. 4**). Two peaks at 3289 cm⁻¹ and 3123 cm⁻¹ are asymmetric and symmetric stretching bonds of NH₂ in amide functional group. Alkene and aromatic C-H stretching vibration bonds appear around 3005 cm⁻¹. The existence of two peaks at 2922 cm⁻¹ and 2853 cm⁻¹ is attributed to asymmetric and symmetric stretching vibrations of aliphatic C-H. A strong peak at 1744 cm⁻¹ demonstrates the carbonyl of ester groups. The carbonyl of amid functional group appears at 1689 cm⁻¹. The presence of C=C bond is shown by the existence of a peak at 1622 cm⁻¹. Two peaks at 1458 cm⁻¹ and 1379 cm⁻¹ show the bending vibrations of methylene and methyl groups respectively. A broad peak between 1100-1250 cm⁻¹ is attributed to the C-O stretching bond of ester groups. The bands at 677 cm⁻¹, 721 cm⁻¹ and 915 cm⁻¹ show the alkene and aromatic C-H bending vibration bonds.³⁰

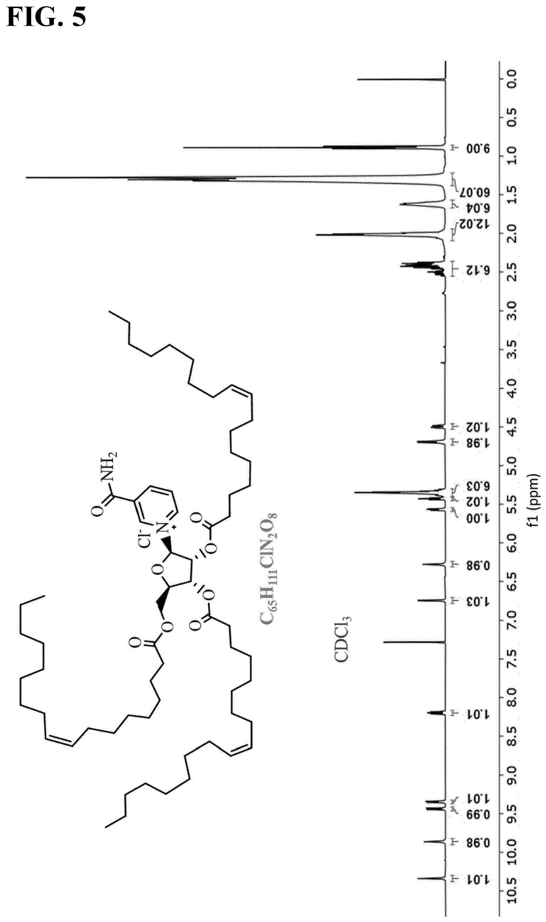

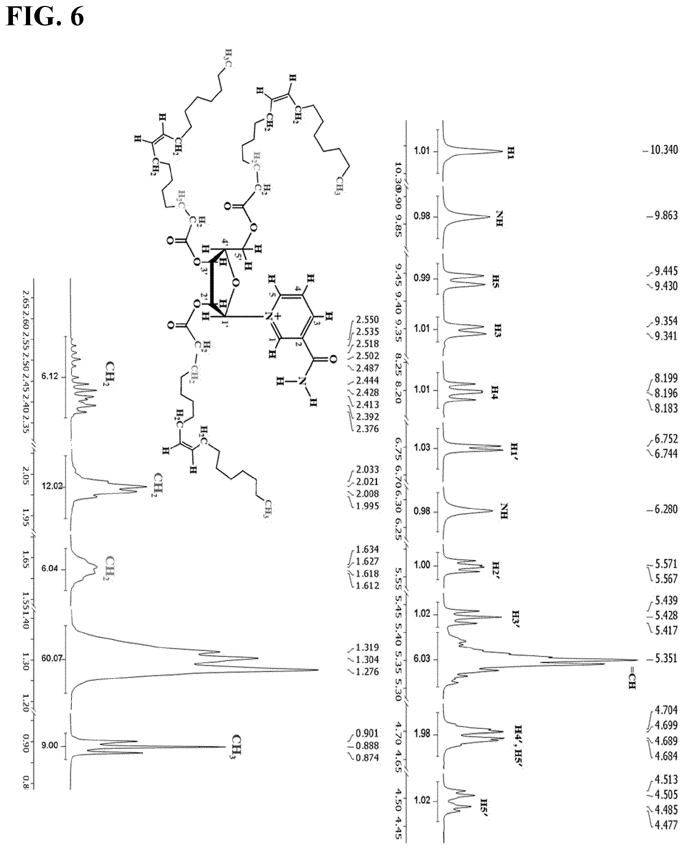

Furthermore, ¹H NMR of NRTOCl was performed in CDCl3 at room temperature, and the integration shows the existence of 111 hydrogens which is in accordance with the structure of this compound (**FIG. 5**). The expanded ¹H NMR of this compound clearly shows that the most deshielded proton (H1) at 10.34 ppm is attributed to the hydrogen located on the pyridinium ring between positive nitrogen and amide group (**FIG. 6**). Because of the conjugation between nitrogen lone pair and carbonyl of amide group,³⁰ the chemical shifts of NH₂ protons are not equivalent in NRTOCl. In this compound, one of the NH₂ protons appears at 9.86 ppm, and another one is at 6.28 ppm. A doublet peak (*J*= 10 Hz) at 9.44 ppm is attributed to H5 located on the pyridinium ring in *ortho* position of positive nitrogen. The chemical shift of H3 in *para* position of positive nitrogen appears 9.34 ppm as a doublet peak (*J*= 10 Hz). The final hydrogen on the pyridinium ring is H4 that appears as a triplet peak (*J* = 10 Hz) at 8.20 ppm. In the structure of NRTO, there are four hydrogens on the ribose ring. The anomeric hydrogen (H1') is impacted more by the oxygen atom in ribose ring and positive nitrogen of pyridinium ring so that this hydrogen appears at 6.75 ppm as a doublet peak (*J* = 5 Hz). H2' and H3' are neighbor groups and appear as two triplet peaks (*J =* 5 Hz) with the chemical shifts of 5.57 and 5.43 ppm respectively. Since H2' is closer to the anomeric center than H3', its chemical shift is more deshielded than that of H3'. A multiplet peak at 5.35 ppm with integral of 6, confirms the presence of three H-C=C-H groups in the structure of NRTO. H4' in the ribose ring and one of the hydrogens of the methylene group (H5') bonded to the single oxygen of ester group overlap with each other and appear as a multiplet peak at 4.70 ppm with integral 2. Since the hydrogens of this methylene group are diastereotopic, another hydrogen of this methylene group appears at 4.50 ppm as a doublet of doublet peak (*J₁=* 14 Hz, *J₂* = 4 Hz). In three fatty ester chains of NRTO, three CH2 groups in the vicinity of ester carbonyl groups appear as a multiplet peak between 2.37-2.55 ppm (**FIG. 6**). Moreover, six methylene groups in the proximity of H-C=C-H groups appear at 2.02 ppm as a multiplet peak. The other three methylene groups in the neighborhood of those CH₂ groups bonded to the carbonyl groups appear at 1.63 ppm as a multiplet peak. A broad peak at 1.30 ppm with integral of 60 are attributed to the rest of 30 methylene groups. Finally, a triplet peak (*J*= 7.0 Hz) at 0.89 ppm with integral of 9 confirms the existence of three methyl groups in the structure of NRTO.

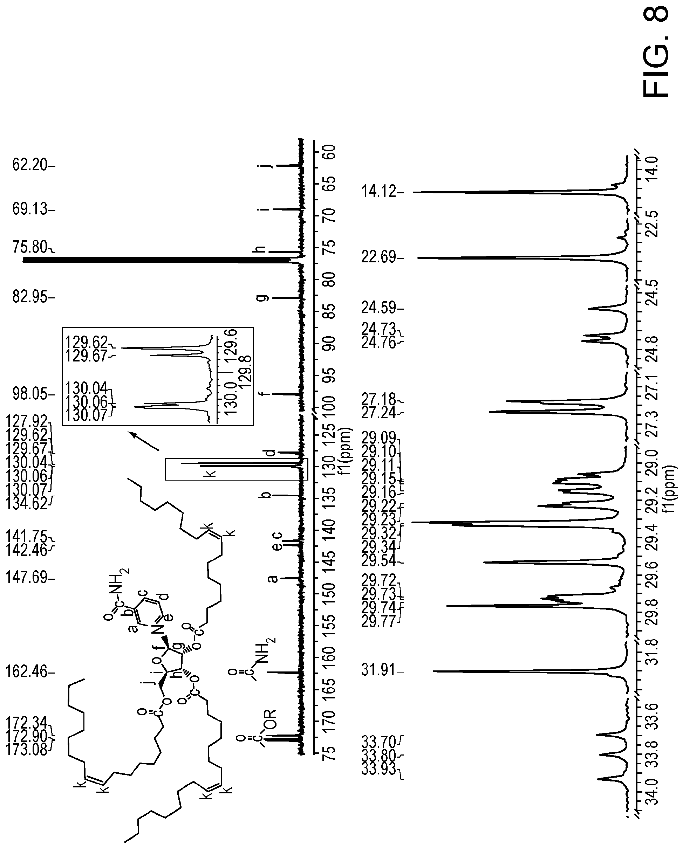

The ¹³C NMR of NRTOCl in CDCl₃ was also studied at room temperature (**FIG**. **7**). The expanded ¹³C NMR of this compound discloses three peaks at 173.1, 172.9 and 172.3 ppm attributed to the three different carbonyl of ester functional groups in the structure of NRTO (**FIG. 8**).

A peak at 162.5 ppm confirms the carbonyl of amide group in this compound. There are five distinct peaks at 146.7, 142.5, 141.8, 134.6 and 127.9 ppm that demonstrate the existence of carbons in the pyridinium ring. The chemical shifts of six carbons of three -C=C-groups are so close together and appear at 130.07, 130.06, 130.04, 129.67 and 129.62 ppm. Due to the close chemical shifts of these carbons, two carbons overlap with together (may be at 129.62 ppm with higher intensity), and consequently, five peaks for these alkene groups were observed. Four peaks at 98.0, 82.9, 75.8 and 69.1 ppm completely demonstrated the existence of a ribose ring in the structure of NRTO. The chemical shift of methylene bonded to single oxygen of the ester group appears at 62.2 ppm. In the three fatty ester chains of NRTO, three distinct peaks at 33.9, 33.8 and 33.7 ppm are attributed to the three CH₂ groups in near-by carbonyl of ester groups (**FIG. 8**). The rest of methylene groups in these chains appear between 22.7-31.9 ppm and, because the chemical shifts of these carbons are close to each other, most of them overlapped together. An intensive peak at 14.1 ppm is attributed to three methyl groups overlapped together.

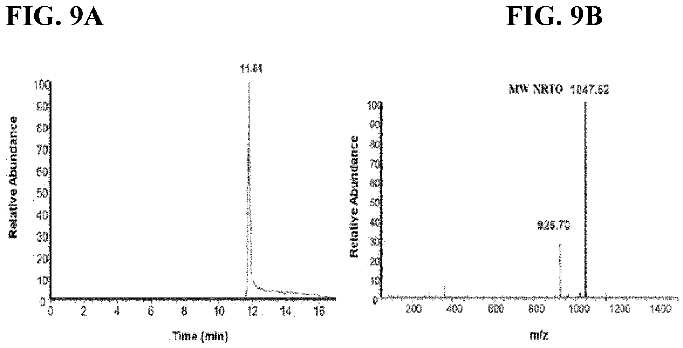

For further assurance, LC-MS study of this compound was performed to find its molecular weight (**FIGS. 9A** and **9B**). The results of selected reaction monitoring (SRM) show a single peak with 1047.52 m/z (M-Cl) that is accurately in agreement with the structure of NRTO. A fragment with 925.70 m/z is attributed to the ribose-trioleates molecule formed by elimination of nicotinamide molecule from NRTO. As a whole result, the obtained spectral data of FTIR, ¹H NMR, ¹³C NMR and LC-MS completely approve the structure of the synthesized NRTOCl by the present procedure.

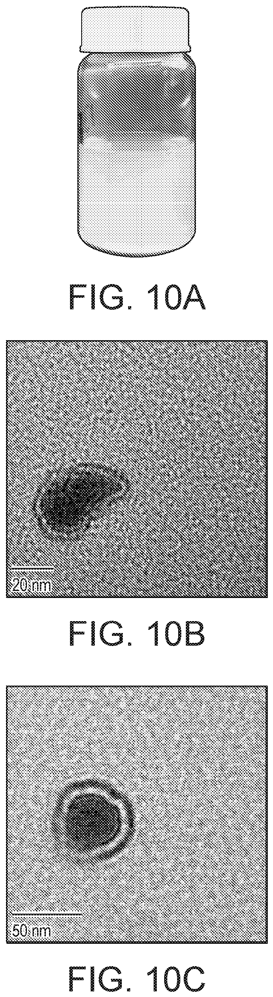

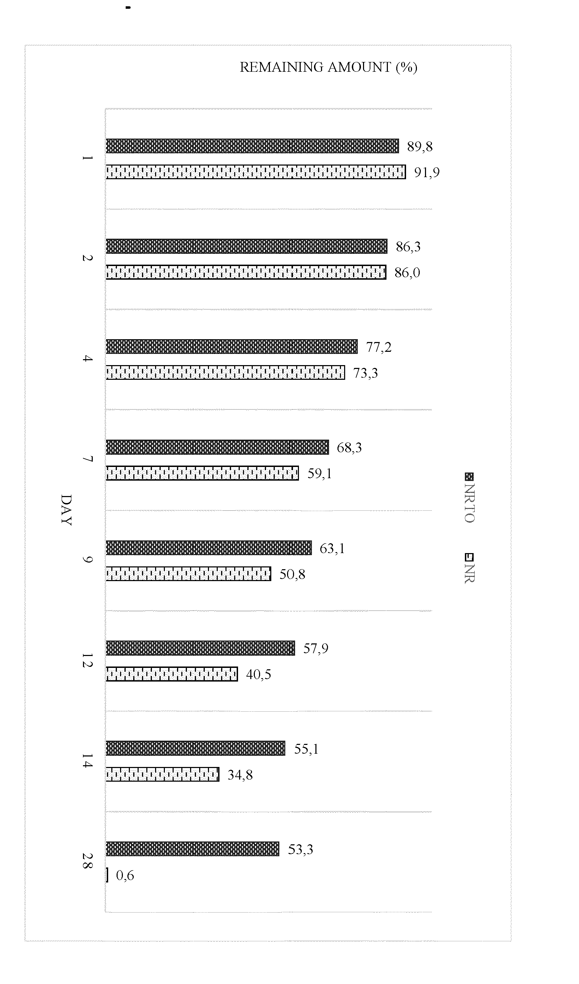

Although NRTOCl is a quaternary ammonium salt, it was not dissolved in water due to the existence of three groups of oleate fatty ester in its structure. Therefore, for the stability study of NRTOCl, this compound was dispersed in DI water using 1 % ethanol as a cosolvent. For this purpose, 15 mg of NRTOCl was dissolved in 0.15 ml of ethanol (1 %) and then 14.85 ml of DI water was added to the mixture and gently shaken. The concentration of NRTOCl in this mixture was 1,000 ppm, and the particle size and the zeta potential were 192 nm and +65 mV respectively. Although the average size of NRTOCl in this sample was 192 nm, the images of TEM disclosed smaller particles with around 50 nm and oval and spherical shapes with aspect ratio close to unity (**FIGS. 10A-10C**). In the nanoparticles, the NRTOCl structures are stacked on top of each other layer-by-layer. The highly positive charge of NRTO makes the nanoparticles stable in the aqueous phase.

After dispersion of NRTOCl in DI water, NRCl was dissolved in DI water, and the stability of these samples were studied at 35 ° C for 28 days (**FIG. 11**). The concentration of each sample was 1000 ppm.

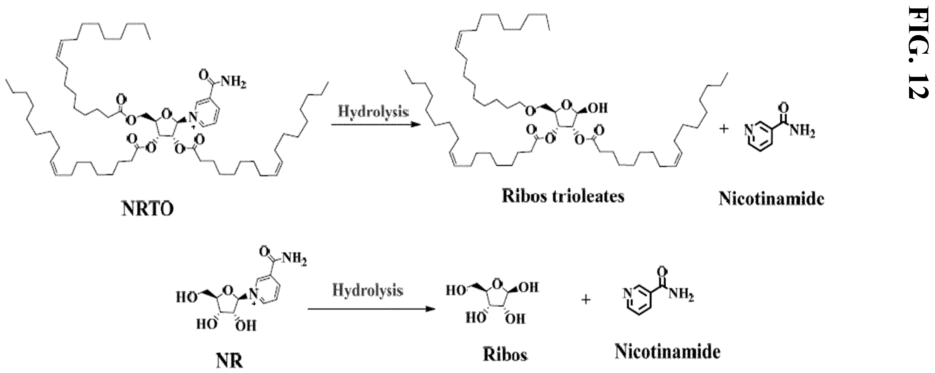

As during the hydrolysis reaction of NRTO and NR, both of these compounds released nicotinamide (**FIG. 12**), and by measuring the released amount of nicotinamide in each sample with HPLC, the remaining amounts of NRTO and NR in each sample were calculated. During this study, any release of NR from NRTO sample was not detected. It signifies that the heavy ester groups in NRTO sample are not hydrolyzed during the time of stability study. Because this study was performed in DI water and the conditions were mild, the NRTO was hydrolyzed from its *N*-glycoside bond.

As shown in **FIG. 11,** the results show that NRTO is more stable than NR in DI water at 35 °C, so that after 28 days the remaining amount of NRTO is 53.3 %, while this amount for NR is 0.6 %. By tri-functionalization of NR with long chain ester groups, the hydrophobicity of this cation increases, and consequently the water accessibility to N-glycosidic bond decreased. After around 42 % hydrolysis of NRTO, the slope of the hydrolysis diagram declines so that after 28 days the remaining amount of NRTO is 53.3 %.

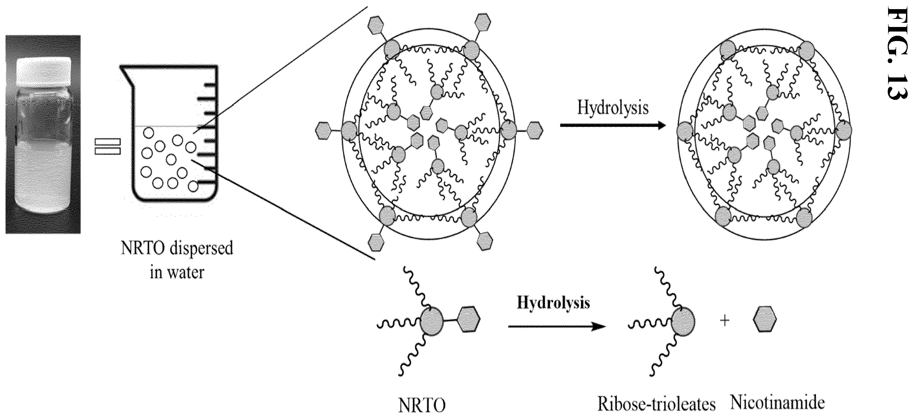

As shown in **FIG. 12****,** ribose trioleates is formed during the hydrolysis process as much as the hydrolyzed NRTO, and structurally this molecule is more hydrophobic than NRTO. Because the hydrolysis process of dispersed NRTO particles is carried out from the outer layers, these layers are gradually converted to ribose trioleates and act as a super hydrophobic shell to minimize the penetration of water into inner layer (**FIG. 13**).

Since NRTOCl is a very hydrophobic compound, to evaluate its stability in oil phase, an emulsion system was used to study the stability of NRTOCl as a function of time. For this purpose, a 15 % w/w solution of NRTOCl in canola oil was used for making different emulsions with Na-caseinate, Tween 80 and lecithin as the emulsifiers (2 wt.%) at room temperature.

When Na-caseinate was used as an emulsifier, the aqueous phase must be a solution of NaCl (0.1 wt.%), CaCl₂ (0.2 wt.%) and KCl (0.3 wt.%) because caseinate anion with minus charge and NRTO with positive charge immediately aggregated in DI water. The total oil phase in these emulsions were 3.2 wt.%, and the concentration of NRTOCl in total volume of each emulsion (15 mL) was 4.4 mM. In the emulsions that used Na-caseinate as an emulsifier, NaN₃ (0.01 wt.%) was added to prevent the growth of bacteria. When the NRTOCl emulsion was made by 2 wt.% Na-caseinate, its average size and zeta potential in this emulsion (Cas emulsion) were 1020 nm and -14.4 mV respectively. These results confirm that the droplets of NRTO in oil phase are completely surrounded by caseinate anions, because the positive charge of NRTO on the surface of droplets becomes the negative value.

By using 2 wt.% Na-caseinate and 2 wt.% lecithin simultaneously as the emulsifiers, the average size and zeta potential of this NRTO emulsion (Cas-Lac emulsion) were 1012 nm and -13.3 mV. An NRTO in canola oil-in-water emulsion was also prepared, using 2 wt.% Tween 80 as an emulsifier in DI water (Tween emulsion). The corresponding average size and zeta potential of this emulsion were 531 nm +49.2 mV respectively. As expected by using Tween 80 as a neutral emulsifier, the positive charge of NRTO droplets was almost intact in the emulsion.

After making NRTOCl emulsions, a NRCl in canola oil-in-water emulsion was attempted as a control experiment using 3.2 % canola oil. The aqueous phase was a solution of Na-caseinate (2 wt.%), NaCl (0.1 wt.%), CaCl₂ (0.2 wt.%), KCl (0.3 wt.%) and NaN₃ (0.01 wt.%). The concentration of NRCl in total volume (15 mL) of this emulsion was 4.4 mM. Moreover, 15 mL of NRCl solution in DI water was prepared with 4.4 mM concentration as the other control experiment. After preparing three NRTOCl emulsions and two control experiment samples, hydrolysis stability of each sample was studied at 35 °C for 26 days (**FIG**. **14**). The rate of degradation was measured by determination of the released nicotinamide from each sample. After 26 days, the remaining amount of NRTO in Cas, Cas-Lec, and Tween emulsions was 93.7, 90.3 and 80.0% respectively. However, this amount for the NR emulsion is 0.4 % and for NR in DI water was 5.3%.

The results demonstrated the stability of NRTO in all emulsions was much better than that of NR in emulsion and in water. The stability of NR in emulsion is less than that of NR in DI water, which shows that NR cannot be dissolved in the oil phase and is only in the aqueous phase. Moreover, since there are several anions and nucleophiles in aqueous phase of the emulsion, the rate of NR hydrolysis increases. Compared to the Tween emulsion, the Cas emulsion and the Cas-Lec emulsion showed better results of stability, so that more than 90 % NRTO was intact in these samples during 26 days. This result means that Na-caseinate as an emulsifier, in comparison to Tween 80, could better act to stabilize the NRTO droplets in the aqueous phase. As already mentioned, caseinate anions can completely surround the surface of NRTO droplets and neutralize the positive charge of NRTO on the outer surface of droplets. This effect may cause the tendency between water lone pair electrons and NRTO decreases in the inter phase. The other factor that can affect the stability of NRTO in these emulsions is the size of the droplets. For the Cas emulsion and the Cas-Lec emulsion, the average size of droplets is almost equal and approximately twice the average size of droplets in the Tween emulsion. This result means that the surface area of the droplets in the Cas and Cas-Lec emulsions was lower than that in Tween emulsion. Therefore, the accessibility of water to the NRTO droplets in the Cas and Cas-Lec emulsions was lower than that of Tween emulsion, and consequently, the rate of the NRTO hydrolysis in these emulations is lower than Tween emulsion.

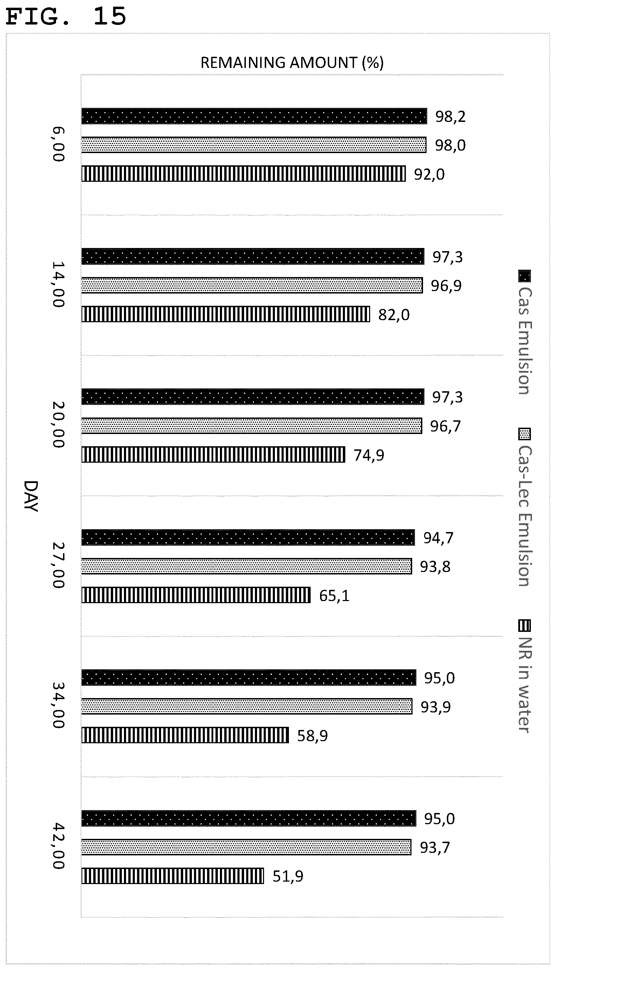

Because NRTO in Cas and Cas-Lec emulsions showed better results of stability at 35 ° C, the stability of NRTO in these emulsions at room temperature was studied for longer time (**FIG. 15**). After 42 days, the remaining amount of NRTO was 95.0 % in the Cas emulsion and 93.7 % in the Cas-Lec emulsion. However, during this time of stability study, 52.0 % NR was intact in water at room temperature. This result means that the rate of NRTO hydrolysis in the emulsions were negligible. During this time, these emulsions were stable without visible phase separation. A little increase in the average size of the NRTO droplets occurred in these emulsions, so that in Cas emulsion, the average size increased from 1020 nm to 1281 nm, and in Cas-Lec emulsion, this parameter increased from 1012 nm to 1106 nm. During all NRTO stability conditions, no released NR from NRTO was measured to confirm the hydrolysis of ester functional groups. As a whole result of stability, NRTO in canola oil-in-water emulsions are much more stable than dispersed NRTO in water and NR in water.

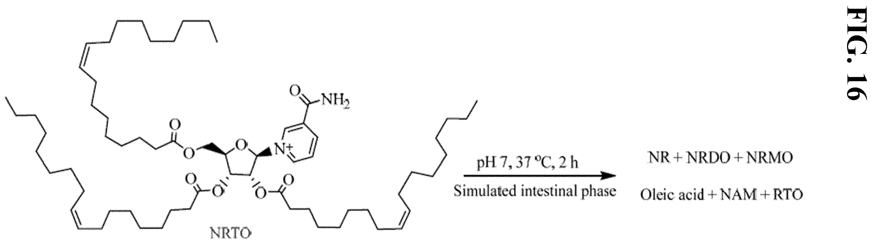

After the NRTOCl stability study in different conditions, the digestibility of this compound to produce NR in simulated intestinal fluid was studied. This enzymatic digestion used porcine pancreatin, bile bovine, and a buffer solution at pH around 7.²⁹ The lipase enzyme in porcine pancreatin can hydrolyze the fatty ester groups to produce NR, NRDO (nicotinamide riboside dioleates), NRMO (nicotinamide riboside monoleate) and oleic acid as the main products of digestion (**FIG. 16**). Moreover, nicotinamide (NAM) and ribose-trioleates (RTO) can be formed as the products of hydrolysis of NRTO not digestion. By considering this hypothesis, the NRTO digestion was studied in simulated intestinal phase.

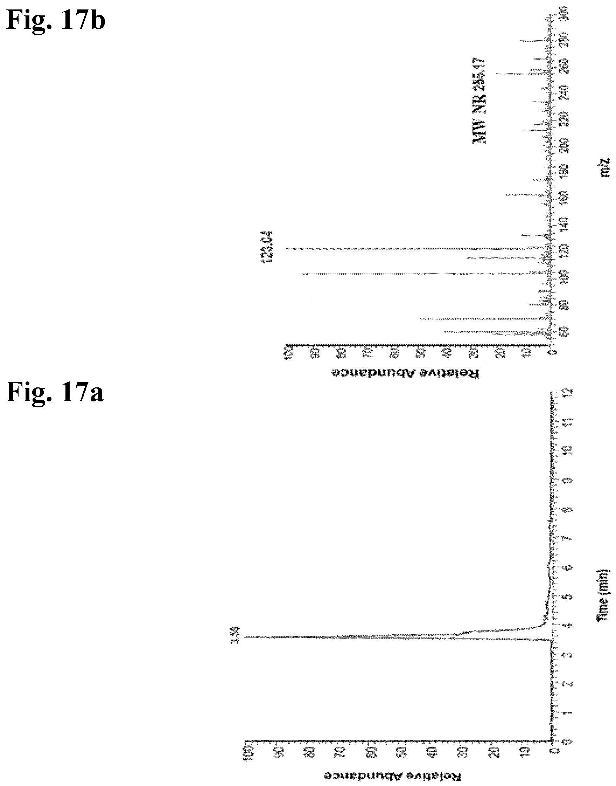

At first, the digestion study was followed by dissolving pure NRTOCl (60 mg) in 0.3 mL of ethanol, and then this solution was added to 10 mL of buffer solution containing 400 mg of bile bovine to disperse NRTO in bile bovine solution. After that, 0.75 mL of CaCl₂ solution (0.3M) was added to this mixture, and the pH was adjusted to 7.0 by adding HCl (1 M). Finally, 400 mg of porcine pancreatin dispersed in 4 mL of buffer solution was added to the mixture, and this sample was placed in an incubator at 37 ° C for 2 hours. The use of 400 mg of bile bovine and 400 mg of porcine pancreatin were the optimized amounts for the enzymatic digestion of NRTO. The released NR and nicotinamide from the sample was measured by LC-MS and HPLC analyses. The results of SRM LC-MS show a single peak with 255.17 m/z (M-Cl) that is accurately in agreement with the structure of NR (**FIGS. 17A** and **17B**). In the mass spectrum, a fragment with 123.04 m/z is attributed to the nicotinamide molecule formed by elimination of ribose molecule from NR. The results showed that after 2 hours of NRTO digestion study, 27.5 % NR was released from this sample. This result means that at least 27.5 % NRTO is completely digested under the reaction conditions.

As a side reaction, 2.4 % nicotinamide was measured from this sample that demonstrated the hydrolysis of *N*-glycosidic bond occured during the NRTO digestion process, but this amount of hydrolysis is low and almost negligible. The other product of NRTO hydrolysis was ribose-trioleates formed in the same amount of nicotinamide (2.4%) during the digestion process. This by-product was not dissolved in aqueous phase to measure it directly. NRMO and NRDO can be the other products of NRTO digestion. However, because we did not have any standard of NRMO and NRDO, we were not able to measure the released amount of these compounds. NRMO was even detected in the aqueous phase by LC-MS with 519.35 m/z but could not be measured, due to the unavailability of its standard.

This study was aimed at digestibility and bioavailability of NRTO to release NR in simulated intestinal phase. The result of 27.5 % released NR shows the digestibility of NRTOCl as a new valuable compound.

To extend the aim of this work, the digestion of NRTO in oil phase was studied. For this purpose and to increase the solubility of NRTO, MCT oil was used instead of canola oil. First, 60 mg of NRTOCl was dissolved in 150 mg of MCT oil to prepare around 29 % (w/w) NRTOCl in MCT oil. Then, the oil phase was added to 10 mL of buffer solution containing 400 mg of bile bovine, and the mixture was homogenized at 15000 rpm for 150 s at room temperature. Next, 0.75 mL of CaCl₂ solution (0.3M) was added to this mixture, and the pH was adjusted to 7.0 by adding HCl (1 M). After that, 400 mg of porcine pancreatin dispersed in 5 mL of buffer solution was added to the mixture, and the sample was placed in an incubator at 37 ° C for 2 hours. The released NR and nicotinamide from this sample were measured by LC-MS and HPLC analyses. Compared to pure digestion of NRTOCl, the released NR from this sample was lower (11.3%). This result was expectable because most of oil phase was MCT oil, and consequently, the accessibility of lipase molecules to NRTO decreases. As the product of N-glycosidic bond hydrolysis, 3.9% nicotinamide was detected during the digestion of NRTO in MCT oil. This porcine pancreatin contained several enzymes including trypsin, chymotrypsin, α-amylase, lipase and colipase. Therefore, the porcine pancreatic lipase is not pure to have high activity. However, the use of the porcine pancreatin and bile bovine at pH 7.0 is one of the best methods to simulate the digestion of lipids in intestinal phase.²⁹ All obtained results confirmed digestibility of NRTO (in pure form and in MTC oil) to produce NR in simulated intestinal phase.

### 3. Conclusion

NRTOCl was synthesized as a new hydrophobic NRCl derivative. The synthesis of NRTOCl was carried out by the reaction between NRCl and oleoyl chloride in the presence of pyridine. The pure product was obtained in 64.3 %, and the results of ¹H NMR, ¹³C NMR, FTIR, and LC-MS confirmed completely the structure of NRTOCl and its purity as well. Because of the presence of three fatty esters in the structure of NRTOCl, it was water insoluble. However, by using EtOH as a cosolvent (1 %), this molecule was dispersed in water as the nanoparticles (average 192 nm) with layer-by-layer structures. The stability of NRCl and NRTOCl in water at 35 °C for 28 days was studied, and the results showed that NRTO was more than 88 times more stable than NRCl. NRTOCl was easily dissolved in canola, corn and MCT oils at room temperature, contrary to NRCl. This feature of NRTOCl helped to evaluate its stability in oil phase by making canola oil-in-water emulsions in the presence of sodium caseinate, lecithin and Tween 80 as the emulsifiers. The stability of NRTO extremely increased in canola oil-in-water emulsion by using sodium caseinate (2 wt.%) as a food grade emulsifier, so that in this system after 26 days and at 35 °C, the unchanged NRTO and NR were 93.7 % and 0.4 % respectively. These findings demonstrated that NRTO was about 213 times more stable than NR in this emulsion system. Also, the stability of NRTO in this canola oil-in-water emulsion system at room temperature for 42 days was studied. These results of stability verified that the hydrolysis of NRTO was negligible (5 %), and it was almost unchanged, while 48 % NR was hydrolyzed during this time. Finally, the bioavailability of this compound was investigated by studying its digestibility in simulated intestinal phase. The results demonstrated that NRTOCl was digestible to release NR in the presence of porcine pancreatin in simulated intestinal phase. The obtained results of stability and digestibility show that NRTOCl has great potential to be used as a NR booster in ready-to-drink (RTD) beverages.

### Example 2

### General procedure for the synthesis of NR-tributyrates chloride (NRTBCl)

To a round bottom flask in ice bath, 300 mg of NRCl, 25 mg of 4-dimethylamino pyridine (DMAPY), 1.5 mL of butyric anhydride, and 9 mL of CH₃CN were added to a round bottom flask and stirred for 5 hours under nitrogen atmosphere. The progress of the reaction was followed by TLC. Next, the solvent was evaporated by rotary evaporator under reduced pressure and the excess amount of butyric anhydride was washed by *n*-hexane. Finally, the crude product was purified by column chromatography on SiO₂. Eluent was the mixture of CH₃OH (35%) and EtOAc (65%). The purified NR-tributyrates chloride (NRTBCl) was obtained in 71 % (367 mg) as a pale-yellow-colored viscous liquid.

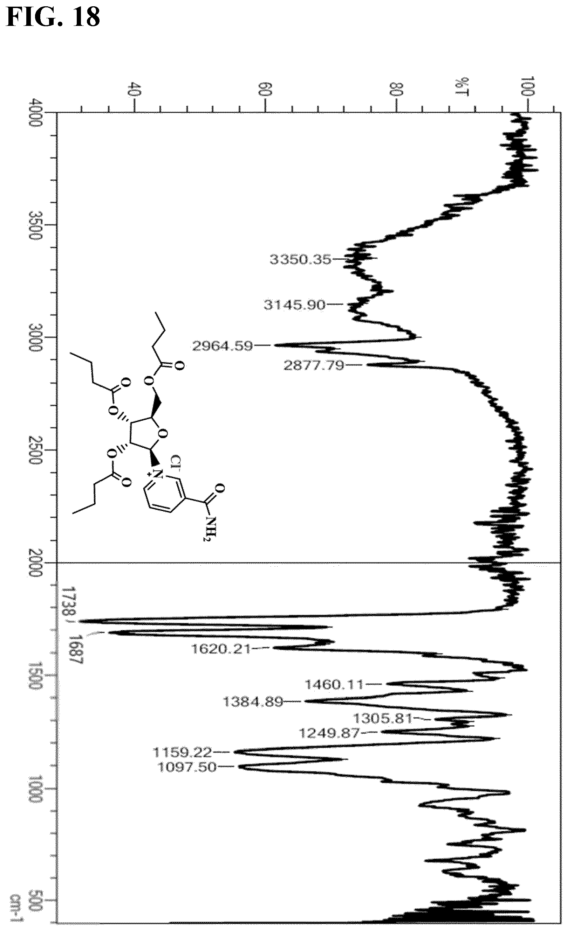

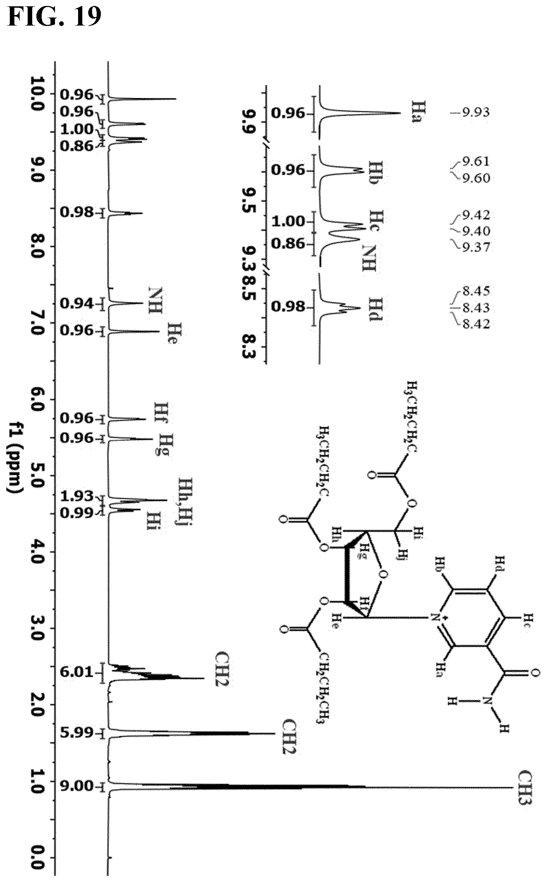

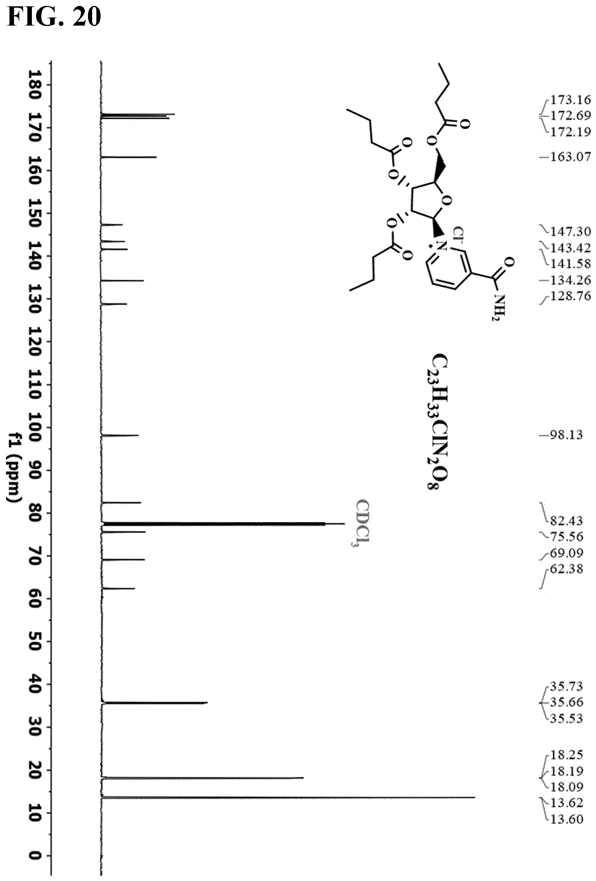

**FIG. 18** shows the FT-IR of NR-tributyrates chloride. **FIG. 19** shows the 1H NMR of NRTBCl in CDCl₃. **FIG. 20** shows the ¹³C NMR of NRTBCl in CDCl₃.

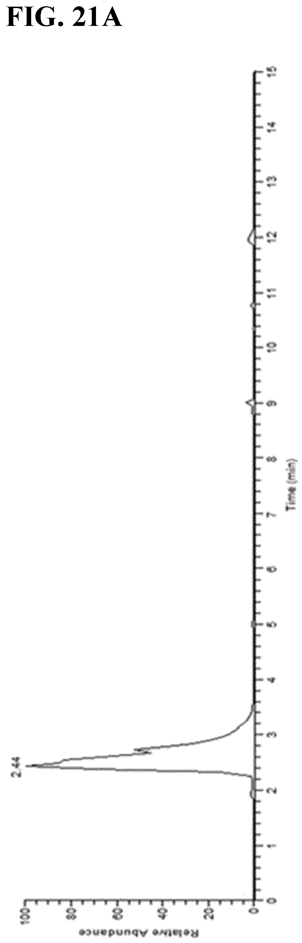

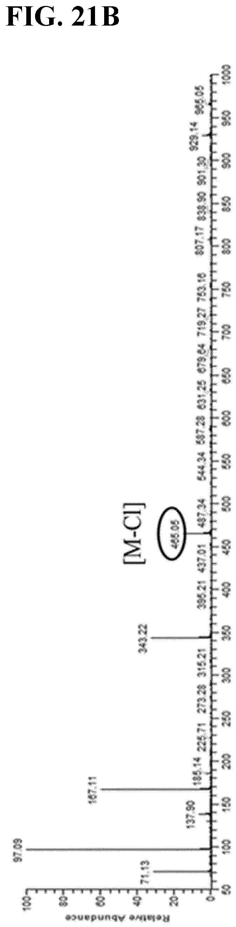

**FIG. 21A** and **21B** show the SRM LC-MS of NRTB. **FIG. 21A** shows SRM LC of NRTB. **FIG. 21A** shows mass spectrum of NRTB.

### Solubility study of NRTBCl in long chain triglycerides

To study the solubility of NRTBCl in long chain triglycerides, we tried to dissolve this compound in olive oil and corn oil. For this purpose, 15 mg of NRTBCl was added to 15 ml of olive oil and vigorously shaked with vortex for 10 minutes at room temperature. The results showed that NRTBCl was not dissolved in olive oil. This procedure was repeated by using corn oil instead of olive oil and the obtained results disclosed that NRTB was insoluble in corn oil.

### Solubility study of NRTBCl in medium chain triglycerides

To study the solubility of NRTBCl in medium chain triglycerides, we tried to dissolve this compound in coconut oil. For this purpose, 15 mg of NRTBCl was added to 15 ml of coconut oil and vigorously shaked with vortex for 10 minutes at room temperature. The results showed that NRTBCl was not dissolved in of coconut oil.

### Solubility study of NRTBCl in water at different pH

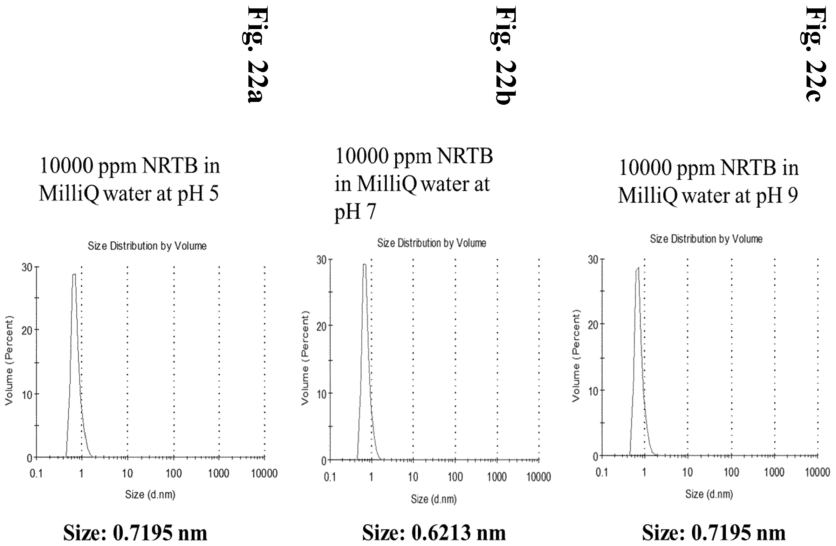

To study the solubility of NRTB in water, three aqueous solutions of NRTBCl were prepared with 10000 ppm concentration at pH 5, 7, and 9. The obtained results of zeta sizer demonstrated that NR-tributyrates can completely be dissolved in water at different pH without forming any aggregation in the solution (**FIGS. 22A, 22B, 22C** which show size measurement of NR-tributyrates chloride in water).

### Preliminary results for NRTBCl stability measurement at 35 °C

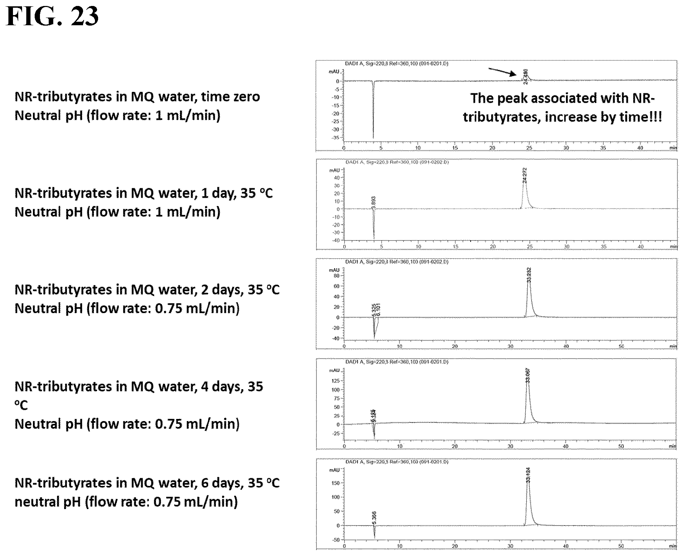

The stability study of the NRTBCl was performed by dissolving NRTB in MQ water and keeping at 35 ° C for 6 days. The concentration of each sample was 1000 ppm. The results of HPLC showed the overlapping of the peak of nicotinamide as a byproduct with the peak of NRTBCl. The reason for increasing the intensity of the peak at 33 min (flow rate: 0.75 mL/min), is that the byproduct of degradation is nicotinamide (NA), which has the retention time of 33 min (exactly as NRTBCl). As shown in **FIG. 23****,** the intensity of the peak after 6 days was obviously higher than that of zero time. It means that NRTBCl can easily hydrolyzed at 35 ° C.

It should be understood that various changes and modifications to the presently preferred embodiments described herein will be apparent to those skilled in the art. Such changes and modifications can be made without departing from the spirit and scope of the present subject matter and without diminishing its intended advantages. It is therefore intended that such changes and modifications be covered by the appended claims.

### References

[1] D. L. Croteau, E. F. Fang, H. Nilsen, V. A. Bohr, Cell Cycle 2017, 16,491.
[2] Y. Yang, A. A. Sauve, Biochimica et Biophysica Acta (BBA)-Proteins and Proteomics 2016, 1864, 1787-1800.
[3] E. F. Fang, V. A. Bohr, Autophagy 2017, 13, 442-443.
[4] E. F. Fang, H. Kassahun, D. L. Croteau, M. Scheibye-Knudsen, K. Marosi, H. Lu, R. A. Shamanna, S. Kalyanasundaram, R. C. Bollineni, M. A. Wilson, Cell metabolism 2016, 24, 566-581.
[5] M. Scheibye-Knudsen, S. J. Mitchell, E. F. Fang, T. Iyama, T. Ward, J. Wang, C. A. Dunn, N. Singh, S. Veith, M. M. Hasan-Olive, Cell metabolism 2014, 20, 840-855.
[6] C. C. Chini, M. G. Tarragó, E. N. Chini, Molecular and cellular endocrinology 2017, 455, 62-74.
[7] H. Zhang, D. Ryu, Y. Wu, K. Gariani, X. Wang, P. Luan, D. D' Amico, E. R. Ropelle, M. P. Lutolf, R. Aebersold, Science 2016, 352, 1436-1443.
[8] M. S. Bonkowski, D. A. Sinclair, Nature reviews Molecular cell biology 2016, 17, 679.
[9] D. W. Frederick, E. Loro, L. Liu, A. Davila Jr, K. Chellappa, I. M. Silverman, W. J. Quinn III, S. J. Gosai, E. D. Tichy, J. G. Davis, Cell metabolism 2016, 24, 269-282.
[10] S. Imai, L. Guarente, Trends in cell biology 2014, 24, 464-471.
[11] N. Braidy, Y. Liu, Current Opinion in Clinical Nutrition & Metabolic Care 2020, 23, 413-420.
[12] C. V. Haar, T. C. Peterson, K. M. Martens, M. R. Hoane, Clin. Pharmacol. Biopharmaceut. S 2013, 1, 1-8.
[13] P. H. Ear, A. Chadda, S. B. Gumusoglu, M. S. Schmidt, S. Vogeler, J. Malicoat, J. Kadel, M. M. Moore, M. E. Migaud, H. E. Stevens, Cell reports 2019, 26, 969-983. e4.
[14] N. Sinthupoom, V. Prachayasittikul, S. Prachayasittikul, S. Ruchirawat, V. Prachayasittikul, European Food Research and Technology 2015, 240, 1-17.
[15] K. Brown, A. Sauve, S. Jaffrey, Use of Nicotinamide Riboside to Treat Hearing Loss, Google Patents, 2018.
[16] G. Ganapati, A. S. R. Arvind, Nicotinamide Riboside Derivatives and Their Uses, Google Patents, 2018.
[17] C. R. Martens, B. A. Denman, M. R. Mazzo, M. L. Armstrong, N. Reisdorph, M. B. McQueen, M. Chonchol, D. R. Seals, Nature communications 2018, 9, 1-11.
[18] N. Djouder, Molecular & cellular oncology 2015, 2, e1001199.
[19] T. Yang, N. Y.-K. Chan, A. A. Sauve, Journal of medicinal chemistry 2007, 50, 6458-6461.
[20] R. Cerutti, E. Pirinen, C. Lamperti, S. Marchet, A. A. Sauve, W. Li, V. Leoni, E. A. Schon, F. Dantzer, J. Auwerx, Cell metabolism 2014, 19, 1042-1049.
[21] B. Gong, Y. Pan, P. Vempati, W. Zhao, L. Knable, L. Ho, J. Wang, M. Sastre, K. Ono, A. A. Sauve, Neurobiology of aging 2013, 34, 1581-1588.
[22] C. Cantó, R. H. Houtkooper, E. Pirinen, D. Y. Youn, M. H. Oosterveer, Y. Cen, P. J. Fernandez-Marcos, H. Yamamoto, P. A. Andreux, P. Cettour-Rose, Cell metabolism 2012, 15, 838-847.
[23] O. L. Dollerup, S. A. Trammell, B. Hartmann, J. J. Holst, B. Christensen, N. Møller, M. P. Gillum, J. T. Treebak, N. Jessen, The Journal of Clinical Endocrinology & Metabolism 2019, 104, 5703-5714.
[24] O. L. Dollerup, B. Christensen, M. Svart, M. S. Schmidt, K. Sulek, S. Ringgaard, H. Stødkilde-Jørgensen, N. Møller, C. Brenner, J. T. Treebak, The American journal of clinical nutrition 2018, 108, 343-353.
[25] H. J. Lee, Y.-S. Hong, W. Jun, S. J. Yang, Journal of medicinal food 2015, 18, 1207-1213.
[26] M. Mehmel, N. Jovanović, U. Spitz, Nutrients 2020, 12, 1616.
[27] Y. Zhou, B. Fu, X. Zheng, D. Wang, C. Zhao, Y. Qi, R. Sun, Z. Tian, X. Xu, H. Wei, National Science Review 2020, 7, 998-1002.
[28] M. T. Campbell, D. S. Jones, G. P. Andrews, S. Li, Food & nutrition research 2019, 63.
[29] A. Brodkorb, L. Egger, M. Alminger, P. Alvito, R. Assunção, S. Ballance, T. Bohn, C. Bourlieu-Lacanal, R. Boutrou, F. Carrière, Nature protocols 2019, 14, 991-1014.
[30] R. M. Silverstein, F. X. Webster, D. J. Kiemle, D. L. Bryce, Spectrometric Identification of Organic Compounds, Wiley; n.d.

## Claims

1. Nicotinamide riboside trioleates chloride (NRTOCl).

2. A composition comprising nicotinamide riboside trioleates chloride (NRTOCl) and optionally at least one of protein, lipid, carbohydrate, vitamin or mineral.

3. The composition of Claim 2, wherein the composition is formulated for oral administration, and preferably is a beverage, such as a Ready-to-Drink (RTD) beverage sealed in a container or a powder formulated for reconstitution in a diluent to form a reconstituted beverage.

4. A unit dosage form of a composition comprising nicotinamide riboside trioleates chloride (NRTOCl), the unit dosage form comprising an amount of the NRTOCl that is therapeutically or prophylactically effective for an individual to whom the unit dosage form is administered.

5. A method of making a composition, the method comprising adding nicotinamide riboside trioleates chloride (NRTOCl) to at least one other component.

6. The method of Claim 5, wherein the composition is formulated for oral administration, and the at least one other component is edible.

7. Nicotinamide riboside trioleates chloride (NRTOCl), the composition according to any one of claims 2-3 or the unit dosage form according to claim 4 for use as a medicament.

8. Nicotinamide riboside trioleates chloride (NRTOCl), the composition according to any one of claims 2-3 or the unit dosage form according to claim 4 for use in a therapeutic method of promoting an increase of intracellular levels of nicotinamide adenine dinucleotide (NAD+) in cells and tissues, the method comprising administering the nicotinamide riboside trioleates chloride (NRTOCl), the composition according to any one of claims 2-3 or the unit dosage form according to claim 4 to an individual, preferably an individual in need thereof, more preferably an older adult or elderly human.

9. Nicotinamide riboside trioleates chloride (NRTOCl), the composition according to any one of claims 2-3 or the unit dosage form according to claim 4 for use in a method of decreasing at least one of DNA damage or mitochondria damage and/or treating or preventing at least one condition selected from the group consisting of (a) a neurodegenerative condition; (b) overweight or obesity; (c) a cardiovascular disease such as heart disease; (d) one or more of diabetes, hyperinsulinemia, an insulin resistance disorder, or insulin insensitivity; (d) muscle degeneration; (e) a disease or disorder associated with aging; (f) a viral infection such as HIV, hepatitis B, SARS-CoV-2 or COVID-19; (g) stress; (h) a blood clotting disorder; (i) inflammation; (j) cancer; (k) an eye disorder; and (l) flushing, the method comprising administering the nicotinamide riboside trioleates chloride (NRTOCl), the composition according to any one of claims 2-3 or the unit dosage form according to claim 4 to a subject in need thereof or at risk thereof.

10. Nicotinamide riboside trioleates chloride (NRTOCl), the composition according to any one of claims 2-3 or the unit dosage form according to claim 4 for use in a therapeutic method of reducing the weight of a subject and/or preventing weight gain in a subject, the method comprising administering the nicotinamide riboside trioleates chloride (NRTOCl), the composition according to any one of claims 2-3 or the unit dosage form according to claim 4 to a subject in need thereof or at risk thereof.

11. Nicotinamide riboside trioleates chloride (NRTOCl), the composition according to claims 2-3 or the unit dosage form according to claim 4 for use in a method of treating or preventing drug toxicity and/or an adverse drug reaction, the method comprising administering the nicotinamide riboside trioleates chloride (NRTOCl), the composition according to any one of claims 2-3 or the unit dosage form according to claim 4 to a subject in need thereof or at risk thereof.

12. The method of making a composition according to any one of claims 5-6 or the composition for use in a method according to any one of claims 8-11, wherein the composition is an emulsion, preferably an oil-in-water emulsion comprising an oil phase in which at least a portion of the NRTOCl is dispersed and optionally in which an emulsifier such as at least one of sodium caseinate or lecithin is dispersed.

13. The method of making a composition according to claim 12 or the composition for use in a method according to claim 12, wherein the oil phase comprises at least one of canola oil, corn oil, or medium chain triglyceride (MCT) oil, in which at least a portion of the NRTOCl is dispersed.

14. The nicotinamide riboside trioleates chloride (NRTOCl) for use in a method according to any one of claims 8-11, the composition for use in method according to any one of claims 8-13 or the unit dosage form for use in a method according to any one of claims 8-11 wherein the nicotinamide riboside trioleates chloride (NRTOCl), composition or unit dosage form is administered daily to the individual for at least one week.

15. The nicotinamide riboside trioleates chloride (NRTOCl) for use in a method according to any one of claims 8-11, the composition for use in a method according to any one of claims 8-14 or the unit dosage for use in a method according to any one of claims 8-11, wherein the individual is selected from the group consisting of a human infant, a human child, a human adolescent, a human adult, an elderly human, and an animal such as a companion animal.

16. The composition for use in method according to any one of claims 8-15, wherein the composition is orally administered, preferably as a beverage, such as a Ready-to-Drink (RTD) beverage sealed in a container which is opened before administration or a powder formulated for reconstitution in a diluent to form a reconstituted beverage before administration.

17. A non-therapeutic method of reducing the weight of a subject and/or preventing weight gain in a subject, the method comprising administering the nicotinamide riboside trioleates chloride (NRTOCl), the composition according to any one of claims 2-3 or the unit dosage form according to claim 4 to a subject in need thereof or at risk thereof.

## Patentansprüche

1. Nicotinamidribosidtrioleatchlorid (NRTOCl).

2. Zusammensetzung, umfassend Nicotinamidribosidtrioleatchlorid (NRTOCl) und gegebenenfalls mindestens eines von Protein, Lipid, Kohlenhydrat, Vitamin oder Mineralstoff.

3. Zusammensetzung nach Anspruch 2, wobei die Zusammensetzung für eine orale Verabreichung formuliert ist und vorzugsweise ein Getränk ist, wie ein trinkfertiges (RTD) Getränk, das in einem Behälter versiegelt ist, oder ein Pulver, das für eine Rekonstitution in einem Verdünnungsmittel formuliert ist, um ein rekonstituiertes Getränk auszubilden.

4. Einheitsdosierungsform einer Zusammensetzung, umfassend Nicotinamidribosidtrioleatchlorid (NRTOCl), die Einheitsdosierungsform umfassend eine Menge des NRTOCl, die für ein Individuum, dem die Einheitsdosierungsform verabreicht wird, therapeutisch oder prophylaktisch wirksam ist.

5. Verfahren zum Herstellen einer Zusammensetzung, das Verfahren umfassend ein Zugeben von Nicotinamidribosidtrioleatchlorid (NRTOCl) zu mindestens einer anderen Komponente.

6. Verfahren nach Anspruch 5, wobei die Zusammensetzung für die orale Verabreichung formuliert ist und die mindestens eine weitere Komponente essbar ist.

7. Nicotinamidribosidtrioleatchlorid (NRTOCl), Zusammensetzung nach einem der Ansprüche 2 bis 3 oder Einheitsdosierungsform nach Anspruch 4 zur Verwendung als ein Medikament.

8. Nicotinamidribosidtrioleatchlorid (NRTOCl), Zusammensetzung nach einem der Ansprüche 2 bis 3 oder Einheitsdosierungsform nach Anspruch 4 zur Verwendung in einem therapeutischen Verfahren zum Fördern einer Erhöhung von intrazellulären Pegeln von Nicotinamidadenindinucleotid (NAD+) in Zellen und Geweben, das Verfahren umfassend das Verabreichen des Nicotinamidribosidtrioleatchlorids (NRTOCl), der Zusammensetzung nach einem der Ansprüche 2 bis 3 oder der Einheitsdosierungsform nach Anspruch 4 an ein Individuum, vorzugsweise ein Individuum, das dies benötigt, mehr bevorzugt einen älteren Erwachsenen oder älteren Menschen.

9. Nicotinamidribosidtrioleatchlorid (NRTOCl), Zusammensetzung nach einem der Ansprüche 2 bis 3 oder Einheitsdosierungsform nach Anspruch 4 zur Verwendung in einem Verfahren zum Verringern von mindestens einem von DNA-Schäden oder Mitochondrienschäden und/oder zum Behandeln oder Vorbeugen von mindestens einem Zustand, ausgewählt aus der Gruppe bestehend aus (a) einem neurodegenerativen Zustand; (b) Übergewicht oder Adipositas; (c) einer kardiovaskulären Erkrankung wie einer Herzerkrankung; (d) einem oder mehreren von Diabetes, Hyperinsulinämie, einer Insulinresistenzstörung oder Insulinunempfindlichkeit; (d) Muskeldegeneration; (e) einer mit Alterung assoziierten Krankheit oder Störung; (f) einer viralen Infektion wie HIV, Hepatitis B, SARS-CoV-2 oder COVID-19; (g) Stress; (h) einer Blutgerinnungsstörung; (i) Entzündung; (j) Krebs; (k) einer Augenstörung; und (l) Hitzewallungen, das Verfahren umfassend das Verabreichen des Nicotinamidribosidtrioleatchlorids (NRTOCl), der Zusammensetzung nach einem der Ansprüche 2 bis 3 oder der Einheitsdosierungsform nach Anspruch 4 an ein Subjekt, das dies benötigt oder bei dem ein Risiko dafür besteht.

10. Nicotinamidribosidtrioleatchlorid (NRTOCl), Zusammensetzung nach einem der Ansprüche 2 bis 3 oder Einheitsdosierungsform nach Anspruch 4 zur Verwendung in einem therapeutischen Verfahren zum Reduzieren des Gewichts eines Subjekts und/oder zum Verhindern einer Gewichtszunahme bei einem Subjekt, das Verfahren umfassend das Verabreichen des Nicotinamidribosidtrioleatchlorids (NRTOCl), der Zusammensetzung nach einem der Ansprüche 2 bis 3 oder der Einheitsdosierungsform nach Anspruch 4 an ein Subjekt, das dies benötigt oder bei dem ein Risiko dafür besteht.

11. Nicotinamidribosidtrioleatchlorid (NRTOCl), Zusammensetzung nach den Ansprüchen 2 bis 3 oder Einheitsdosierungsform nach Anspruch 4 zur Verwendung in einem Verfahren zum Behandeln oder zum Vorbeugen von Arzneimitteltoxizität und/oder einer unerwünschten Arzneimittelwirkung, das Verfahren umfassend das Verabreichen des Nicotinamidribosidtrioleatchlorids (NRTOCl), der Zusammensetzung nach einem der Ansprüche 2 bis 3 oder der Einheitsdosierungsform nach Anspruch 4 an ein Subjekt, das dies benötigt oder bei dem ein Risiko dafür besteht,.

12. Verfahren zum Herstellen einer Zusammensetzung nach einem der Ansprüche 5 bis 6 oder Zusammensetzung zur Verwendung in einem Verfahren nach einem der Ansprüche 8 bis 11, wobei die Zusammensetzung eine Emulsion ist, vorzugsweise eine Öl-in-Wasser-Emulsion, umfassend eine Ölphase, in der mindestens ein Anteil des NRTOCl dispergiert ist und in der gegebenenfalls ein Emulgator wie mindestens eines von Natriumcaseinat oder Lecithin dispergiert ist.

13. Verfahren zum Herstellen einer Zusammensetzung nach Anspruch 12 oder Zusammensetzung zur Verwendung in einem Verfahren nach Anspruch 12, wobei die Ölphase mindestens eines von Rapsöl, Maisöl oder mittelkettigem Triglyceridöl (MCT-Öl) umfasst, in der mindestens ein Anteil des NRTOCl dispergiert ist.

14. Nicotinamidribosidtrioleatchlorid (NRTOCl) zur Verwendung in einem Verfahren nach einem der Ansprüche 8 bis 11, Zusammensetzung zur Verwendung in einem Verfahren nach einem der Ansprüche 8 bis 13 oder Einheitsdosierungsform zur Verwendung in einem Verfahren nach einem der Ansprüche 8 bis 11, wobei das Nicotinamidribosidtrioleatchlorid (NRTOCl), die Zusammensetzung oder die Einheitsdosierungsform dem Individuum für mindestens eine Woche täglich verabreicht wird.

15. Nicotinamidribosidtrioleatchlorid (NRTOCl) zur Verwendung in einem Verfahren nach einem der Ansprüche 8 bis 11, Zusammensetzung zur Verwendung in einem Verfahren nach einem der Ansprüche 8 bis 14 oder Einheitsdosis zur Verwendung in einem Verfahren nach einem der Ansprüche 8 bis 11, wobei das Individuum ausgewählt ist aus der Gruppe bestehend aus einem menschlichen Säugling, einem menschlichen Kind, einem menschlichen Jugendlichen, einem menschlichen Erwachsenen, einem älteren Menschen und einem Tier wie einem Haustier.

16. Zusammensetzung zur Verwendung in dem Verfahren nach einem der Ansprüche 8 bis 15, wobei die Zusammensetzung oral verabreicht wird, vorzugsweise als ein Getränk, wie ein trinkfertiges (RTD) Getränk, das in einem Behälter versiegelt ist, der vor der Verabreichung geöffnet wird, oder ein Pulver, das für die Rekonstitution in einem Verdünnungsmittel formuliert ist, um vor der Verabreichung ein rekonstituiertes Getränk auszubilden.

17. Nicht therapeutisches Verfahren zum Reduzieren des Gewichts eines Subjekts und/oder zum Verhindern einer Gewichtszunahme bei einem Subjekt, das Verfahren umfassend das Verabreichen des Nicotinamidribosidtrioleatchlorids (NRTOCl), der Zusammensetzung nach einem der Ansprüche 2 bis 3 oder der Einheitsdosierungsform nach Anspruch 4 an ein Subjekt, das dies benötigt oder bei dem ein Risiko dafür besteht.

## Revendications

1. Chlorure de trioléates de nicotinamide riboside (NRTOCl).

2. Composition comprenant du chlorure de trioléates de nicotinamide riboside (NRTOCl) et facultativement au moins l'un parmi protéine, lipide, glucide, vitamine ou minéral.

3. Composition selon la revendication 2, dans laquelle la composition est formulée pour une administration orale, et de préférence est une boisson, telle qu'une boisson prête-à-boire (RTD) scellée dans un récipient ou une poudre formulée pour une reconstitution dans un diluant pour former une boisson reconstituée.

4. Forme posologique unitaire d'une composition comprenant du chlorure de trioléates de nicotinamide riboside (NRTOCl), la forme posologique unitaire comprenant une quantité du NRTOCl qui est efficace sur le plan thérapeutique ou prophylactique pour un individu à qui la forme posologique unitaire est administrée.

5. Procédé de préparation d'une composition, le procédé comprenant l'ajout de chlorure de trioléates de nicotinamide riboside (NRTOCl) à au moins un autre composant.

6. Procédé selon la revendication 5, dans lequel la composition est formulée pour une administration orale, et l'au moins un autre composant est comestible.

7. Chlorure de trioléates de nicotinamide riboside (NRTOCl), composition selon l'une quelconque des revendications 2 à 3 ou forme posologique unitaire selon la revendication 4 pour une utilisation en tant que médicament.

8. Chlorure de trioléates de nicotinamide riboside (NRTOCl), composition selon l'une quelconque des revendications 2 à 3 ou forme posologique unitaire selon la revendication 4 pour une utilisation dans un procédé thérapeutique consistant à favoriser une augmentation de niveaux intracellulaires de dinucléotide nicotinamide-adénine (NAD+) dans des cellules et des tissus, le procédé comprenant l'administration du chlorure de trioléates de nicotinamide riboside (NRTOCl), de la composition selon l'une quelconque des revendications 2 à 3 ou de la forme posologique unitaire selon la revendication 4 à un individu, de préférence un individu en ayant besoin, plus préférablement un être humain adulte d'âge avancé ou âgé.

9. Chlorure de trioléates de nicotinamide riboside (NRTOCl), composition selon l'une quelconque des revendications 2 à 3 ou forme posologique unitaire selon la revendication 4 pour une utilisation dans un procédé consistant à diminuer au moins l'un parmi des dommages à l'ADN ou des dommages aux mitochondries et/ou à traiter ou à prévenir au moins un état choisi dans le groupe constitué de (a) un état neurodégénératif ; (b) surpoids ou obésité ; (c) une maladie cardiovasculaire telle qu'une maladie cardiaque ; (d) un ou plusieurs parmi diabète, hyperinsulinémie, un trouble de la résistance à l'insuline, ou insensibilité à l'insuline ; (d) dégénérescence musculaire ; (e) une maladie ou un trouble associé au vieillissement ; (f) une infection virale telle que VIH, hépatite B, SRAS-CoV-2 ou COVID-19 ; (g) stress ; (h) un trouble de la coagulation sanguine ; (i) inflammation ; (j) cancer ; (k) un trouble oculaire ; et
(l) rougissement, le procédé comprenant l'administration du chlorure de trioléates de nicotinamide riboside (NRTOCl), de la composition selon l'une quelconque des revendications 2 à 3 ou de la forme posologique unitaire selon la revendication 4 à un sujet en ayant besoin ou à risque.

10. Chlorure de trioléates de nicotinamide riboside (NRTOCl), composition selon l'une quelconque des revendications 2 à 3 ou forme posologique unitaire selon la revendication 4 pour une utilisation dans un procédé thérapeutique consistant à réduire le poids d'un sujet et/ou à prévenir une prise de poids chez un sujet, le procédé comprenant l'administration du chlorure de trioléates de nicotinamide riboside (NRTOCl), de la composition selon l'une quelconque des revendications 2 à 3 ou de la forme posologique unitaire selon la revendication 4 à un sujet en ayant besoin ou à risque.

11. Chlorure de trioléates de nicotinamide riboside (NRTOCl), composition selon les revendications 2 à 3 ou forme posologique unitaire selon la revendication 4 pour une utilisation dans un procédé consistant à traiter ou à prévenir une toxicité médicamenteuse et/ou une réaction indésirable à un médicament, le procédé comprenant l'administration du chlorure de trioléates de nicotinamide riboside (NRTOCl), de la composition selon l'une quelconque des revendications 2 à 3 ou de la forme posologique unitaire selon la revendication 4 à un sujet en ayant besoin ou à risque.

12. Procédé de préparation d'une composition selon l'une quelconque des revendications 5 à 6 ou composition pour une utilisation dans un procédé selon l'une quelconque des revendications 8 à 11, dans lequel la composition est une émulsion, de préférence une émulsion huile-dans-eau comprenant une phase huileuse dans laquelle au moins une partie du NRTOCl est dispersée et facultativement dans laquelle un émulsifiant tel qu'au moins l'un parmi caséinate de sodium ou lécithine est dispersé.

13. Procédé de préparation d'une composition selon la revendication 12 ou composition pour une utilisation dans un procédé selon la revendication 12, dans lequel la phase huileuse comprend au moins l'une parmi huile de canola, huile de maïs, ou huile à triglycérides à chaîne moyenne (MCT), dans lequel au moins une partie du NRTOCl est dispersée.

14. Chlorure de trioléates de nicotinamide riboside (NRTOCl) pour une utilisation dans un procédé selon l'une quelconque des revendications 8 à 11, composition pour une utilisation dans un procédé selon l'une quelconque des revendications 8 à 13 ou forme posologique unitaire pour une utilisation dans un procédé selon l'une quelconque des revendications 8 à 11, dans lequel le chlorure de trioléates de nicotinamide riboside (NRTOCl), la composition ou la forme posologique unitaire est administré quotidiennement à l'individu pendant au moins une semaine.

15. Chlorure de trioléates de nicotinamide riboside (NRTOCl) pour une utilisation dans un procédé selon l'une quelconque des revendications 8 à 11, composition pour une utilisation dans un procédé selon l'une quelconque des revendications 8 à 14 ou dose unitaire pour une utilisation dans un procédé selon l'une quelconque des revendications 8 à 11, dans lequel l'individu est choisi dans le groupe constitué d'un nourrisson humain, d'un enfant humain, d'un adolescent humain, d'un adulte humain, d'un être humain âgé, et d'un animal tel qu'un animal de compagnie.

16. Composition pour une utilisation dans un procédé selon l'une quelconque des revendications 8 à 15, dans laquelle la composition est administrée par voie orale, de préférence en tant que boisson, telle qu'une boisson prête-à-boire (RTD) scellée dans un récipient qui est ouvert avant administration ou une poudre formulée pour une reconstitution dans un diluant pour former une boisson reconstituée avant administration.

17. Procédé non thérapeutique consistant à réduire le poids d'un sujet et/ou à prévenir une prise de poids chez un sujet, le procédé comprenant l'administration du chlorure de trioléates de nicotinamide riboside (NRTOCl), de la composition selon l'une quelconque des revendications 2 à 3 ou de la forme posologique unitaire selon la revendication 4 à un sujet en ayant besoin ou à risque.
